(19) Europäisches Patentamt
European Patent Office
Office européen des brevets

(11) **EP 4 806 356 A1**

(12) **EUROPEAN PATENT APPLICATION**
published in accordance with Art. 153(4) EPC

(43) Date of publication:
**16.09.2026 Bulletin 2026/38**

(21) Application number: **24887764.9**

(22) Date of filing: **24.10.2024**

(51) International Patent Classification (IPC):
***A61B 6/00*** *(2024.01)*

(52) Cooperative Patent Classification (CPC):
**G01L 5/16; A61B 6/00; A61B 6/40; A61B 6/42; A61B 6/4405; A61B 6/4441; A61B 6/4452; A61B 6/4464; A61B 6/4476; A61B 6/4482; H05G 1/30**

(86) International application number:
**PCT/CN2024/127069**

(87) International publication number:
**WO 2025/098151 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.11.2023 CN 202311478593**

(71) Applicant: **Shenzhen Mindray Bio-Medical Electronics Co., Ltd.**
**Shenzhen, Guangdong 518057 (CN)**

(72) Inventors:
- **LIU, Xuedong**
  **Shenzhen, Guangdong 518057 (CN)**
- **ZHANG, Zanchao**
  **Shenzhen, Guangdong 518057 (CN)**
- **LI, Jianbo**
  **Shenzhen, Guangdong 518057 (CN)**
- **WANG, Yuan**
  **Shenzhen, Guangdong 518057 (CN)**
- **LI, Ning**
  **Shenzhen, Guangdong 518057 (CN)**
- **YANG, Nengfei**
  **Shenzhen, Guangdong 518057 (CN)**
- **HUANG, Bo**
  **Shenzhen, Guangdong 518057 (CN)**

(74) Representative: **KIPA AB**
**Drottninggatan 11**
**252 21 Helsingborg (SE)**

# (54) X-RAY GENERATING APPARATUS AND X-RAY IMAGING SYSTEM

(57) Disclosed are an X-ray generation device and an X-ray imaging system. The device comprises a suspension assembly, a head assembly, an electric assist assembly, a combined multi-dimensional force sensor, and a controller. The suspension assembly includes a guide rail, a moving member, a lifting arm, and a rotating arm. The head assembly includes a connection base, a bracket, a control head, and a handle. The handle drives the control head with six degrees of freedom including translation along X, Y, Z axes and rotation about the Z-axis, a first direction, and a second direction, enabling the control head to be positioned arbitrarily within its range for imaging patients in various postures and positions. Six electric assist members drive the control head through the six degrees of freedom, allowing medical staff to operate the control head by applying minimal force to the handle, thereby saving time and effort.

10
30
31
11
11
111
112
12
21
13
231
14
22
23
20
24
X-axis direction
Y-axis direction
Z-axis direction
first direction
second direction
third direction

FIG. 1

EP 4 806 356 A1

## Description

### TECHNICAL FIELD

The present disclosure relates to the field of medical detection technology, and specifically, to an X-ray generation device and an X-ray imaging system.

### BACKGROUND OF THE DISCLOSURE

A suspended X-ray imaging system includes an X-ray generation device suspended from the ceiling. The X-ray generation device includes a control head capable of emitting X-rays.

Currently, suspended X-ray imaging systems are primarily designed for imaging patients either standing beside the lifting arm or lying on the flat panel - that is, patients in fixed positions. As a result, existing products offer limited degrees of freedom in the control head, which prevents it from capturing images of patients in different locations or at varying posture angles. Furthermore, due to the substantial weight of the suspended control head, medical staff often find it time-consuming and physically demanding to maneuver.

### SUMMARY OF THE DISCLOSURE

The present disclosure provides an X-ray generation device and an X-ray imaging system for addressing the problems of limited degrees of freedom in movement of the control head and difficulty in operating it.

An X-ray generation device is provided in an embodiment, comprising:

a suspension assembly, comprising a first guide rail, a second guide rail, a moving member, a lifting arm, and a rotating arm, wherein the first guide rail extends along an X-axis direction, the second guide rail extends along a Y-axis direction, the second guide rail is movably connected to the first guide rail and is movable relative to the first guide rail along the X-axis direction, the moving member is movably connected to the second guide rail and is movable relative to the second guide rail along the Y-axis direction, the lifting arm has a first end and a second end that are opposite to each other, the first end of the lifting arm is connected to the moving member, and the second end of the lifting arm is liftable along the Z-axis direction, the rotating arm is connected to the second end of the lifting arm and is rotatable about the Z-axis direction;
a head assembly, comprising a connection base, a bracket, a control head and a handle, wherein the connection base is connected to the rotating arm, and the bracket is rotatably connected to the connection base and is rotatable relative to the connection base about a first direction, the control head generates X-rays and emit them to a region to be examined, the control head is rotatably connected to the bracket and is rotatable relative to the bracket about a second direction, the handle is connected to the control head and is operable by a user to effectuate the movement, lifting, and/or rotation;
an electric assist assembly, comprising a first electric assist member, a second electric assist member, a third electric assist member, a fourth electric assist member, a fifth electric assist member, and a sixth electric assist member, wherein the first electric assist member is connected to the second guide rail so as to drive the second guide rail to move along the X-axis direction, the second electric assist member is connected to the moving member so as to drive the moving member to move along the Y-axis direction, the third electric assist member is connected to the lifting arm so as to drive the lifting arm to lift along the Z-axis direction, the fourth electric assist member is connected to the rotating arm so as to drive the rotating arm to rotate about the Z-axis direction, the fifth electric assist member is connected to the bracket so as to drive the bracket to rotate relative to the connection base about the first direction, the sixth electric assist member is connected to the control head so as to drive the control head to rotate relative to the bracket about the second direction;
a combined multi-dimensional force sensor, connected to the handle, configured to detect force applied to the handle and generate corresponding detection signals; and
a controller, in signal communication with the combined multi-dimensional force sensor, the first electric assist member, the second electric assist member, the third electric assist member, the fourth electric assist member, the fifth electric assist member, and the sixth electric assist member, wherein the controller obtains the detection signals and generate corresponding control signals, and send the control signals to one or more of the first electric assist member, the second electric assist member, the third electric assist member, the fourth electric assist member, the fifth electric assist member, and the sixth electric assist member, so as to control the movement, lifting, and/or rotation of the control head.

In some embodiments, the first electric assist member comprises a first drive motor, a first drive pulley, and a first

drive belt, wherein the first drive motor and the first drive pulley are mounted on the second guide rail, the first drive belt is mounted along the X-axis direction on the first guide rail, an output shaft of the first drive motor is fixedly connected to the first drive pulley, the first drive pulley is engaged with the first drive belt, and the first drive motor drives the first drive pulley to travel relative to the first drive belt along the X-axis direction, thereby causing the second guide rail to move relative to the first guide rail along the X-axis direction;

and/or,

the second electric assist member comprises a second drive motor, a second drive pulley, and a second drive belt, wherein the second drive motor and the second drive pulley are mounted on the moving member, the second drive belt is mounted along the Y-axis direction on the second guide rail, an output shaft of the second drive motor is fixedly connected to the second drive pulley, the second drive pulley is engaged with the second drive belt, the second drive motor drives the second drive pulley to travel relative to the second drive belt along the Y-axis direction, thereby causing the moving member to move relative to the second guide rail along the Y-axis direction.

In some embodiments, the third electric assist member comprises a third drive motor, a first transmission assembly, and a traction element that are mounted on the moving member, the third drive motor is connected to the traction element via the first transmission assembly, the traction element extends to connect to the second end of the lifting arm, and the third drive motor drives, via the first transmission assembly, the traction element to move up and down, thereby driving the second end of the lifting arm to lift along the Z-axis direction.

In some embodiments, the first transmission assembly comprises two third drive pulleys, a third drive belt, and a roller shaft; an output shaft of the third drive motor is fixedly connected to one of the third drive pulleys, with the roller shaft fixedly connected to the other of the third drive pulleys; the two third drive pulleys are engaged with the third drive belt; and the traction element is a traction rope wound around the roller shaft, with one end of the traction rope fixedly connected to the roller shaft, and the other end of the traction rope fixedly connected to the second end of the lifting arm.

In some embodiments, the lifting arm at least comprises a first lifting sub-arm and a second lifting sub-arm, the first lifting sub-arm and the second lifting sub-arm are movably connected for lifting along the Z-axis direction, an end of the first lifting sub-arm away from the second lifting sub-arm is defined as the first end of the lifting arm, an end of the second lifting sub-arm away from the first lifting sub-arm is defined as the second end of the lifting arm, the first lifting sub-arm has a hollow structure, and an end of the traction element extends through the first lifting sub-arm so as to be fixedly connected to the second lifting sub-arm.

In some embodiments, the fourth electric assist member comprises a fourth drive motor, a first helical gear, and a second helical gear, the fourth drive motor is mounted on the rotating arm, an output shaft of the fourth drive motor is fixedly connected to the first helical gear, the second helical gear is fixedly connected to the second end of the lifting arm, the first helical gear is meshed with the second helical gear, a central axis of the second helical gear is parallel to the Z-axis direction, and a central axis of the first helical gear intersects with the central axis of the second helical gear.

In some embodiments, the central axis of the first helical gear is perpendicular to the central axis of the second helical gear.

In some embodiments, the rotating arm has a hollow structure, and the fourth drive motor, the first helical gear and at least a portion of the second helical gear are disposed within the rotating arm.

In some embodiments, the second helical gear has a hollow structure, the second end of the lifting arm is rotatably connected to the rotating arm by a first rotating shaft, and the first rotating shaft extends through a central portion of the second helical gear.

In some embodiments, the suspension assembly further comprises a first limiting structure, the first limiting structure comprises a first limiting member and a second limiting member, the first limiting member is mounted on the second end of the lifting arm, the second limiting member is arranged on the rotating arm, the first limiting member is mounted on a circular path along which the second limiting member rotates, and the first limiting member abuts against the second limiting member, so as to limit an angular range of the rotation of the rotating arm about the Z-axis direction to -180° - +180°.

In some embodiments, the first limiting member comprises a swing member and an angle limiting member; one end of the swing member is rotatably connected to the second end of the lifting arm; the other end of the swing member, defined as a limiting end, blockingly abuts against the second limiting member; the angle limiting member is arranged on a swing path of the swing member and limits a swing angle of the swing member, thereby limiting maximum rotational positions of the rotating arm about the Z-axis direction at -180° and 180°.

In some embodiments, a central portion of the swing member is provided with a first arc-shaped groove or a first arc-shaped hole; a portion of the angle limiting member is disposed in the first arc-shaped groove or the first arc-shaped hole; and the first arc-shaped groove or the first arc-shaped hole has a predetermined arc length for limiting the swing angle of the swing member.

In some embodiments, the suspension assembly further comprises a positioning structure; the positioning structure comprises a first positioning member and a second positioning member; one of the first positioning member and the second positioning member is mounted on the second end of the lifting arm, and the other thereof is mounted on the

rotating arm; the first positioning member is a ring-shaped structure provided with a plurality of positioning holes along a circumference thereof; and an end portion of the second positioning member is provided with a retractable elastic portion configured to be engageable with the positioning holes so as to position a rotational angle of the rotating arm.

In some embodiments, the fifth electric assist member comprises a fifth drive motor and a transmission shaft; the fifth drive motor is mounted on the connection base; one end of the transmission shaft is fixedly connected to an output shaft of the fifth drive motor; the other end of the transmission shaft is fixedly connected to the bracket; and the transmission shaft is parallel to the first direction.

In some embodiments, the head assembly further comprises a second limiting structure; the second limiting structure comprises a third limiting member and two fourth limiting members; the third limiting member is mounted on the transmission shaft; the two fourth limiting members are mounted on the connection base; the two fourth limiting members are arranged on a circular path along which the third limiting member rotates; and the two fourth limiting members abuts against the third limiting member, respectively, so as to limit an angular range of the rotation of the bracket about the first direction to -140° - +140°.

In some embodiments, the sixth electric assist member comprises a sixth drive motor and a second transmission assembly; the sixth drive motor is mounted on the control head; the sixth drive motor is connected to the bracket via the second transmission assembly; and the sixth drive motor drives, via the second transmission assembly, the bracket to rotate about the second direction.

In some embodiments, the bracket and the control head are rotatably connected via a second rotating shaft; the second transmission assembly comprises two fourth drive pulleys and a fourth drive belt; one of the fourth drive pulleys is fixedly connected to an output shaft of the sixth drive motor; the other of the fourth drive pulleys is fixedly connected to the bracket and is arranged coaxially with the second rotating shaft; and the two fourth drive pulleys are engaged with the fourth drive belt.

In some embodiments, the head assembly further comprises a third limiting structure; the third limiting structure comprises a fifth limiting member and a sixth limiting member; one of the fifth limiting member and the sixth limiting member is mounted on the bracket, and the other of the fifth limiting member and the sixth limiting member is mounted on the control head; the fifth limiting member is provided with a second arc-shaped groove or a second arc-shaped hole; a portion of the sixth limiting member is engaged in the second arc-shaped groove or the second arc-shaped hole; the sixth limiting member moves within the second arc-shaped groove or the second arc-shaped hole; and the second arc-shaped groove or the second arc-shaped hole has a predetermined arc length for limiting an angular range of the rotation of the control head about the second direction to -10° - +90°.

In some embodiments, the combined multi-dimensional force sensor comprises a force sensor group; the force sensor group comprises one or more of: a one-dimensional force sensor, a two-dimensional force sensor, and a three-dimensional force sensor; and the force sensor group detects respective forces applied to the handle in the first direction, the second direction, and a third direction, and outputs the detection signals representing movement of the handle along the X-axis direction, movement of the handle along the Y-axis direction, movement of the handle along the Z-axis direction, rotation of the handle about the Z-axis direction, rotation of the handle about the first direction, and/or rotation of the handle about the second direction.

In some embodiments, the force sensor group comprises comprises at least four two-dimensional force sensors; the at least four two-dimensional force sensors are divided into a first group and a second group; the first group and the second group each comprises two two-dimensional force sensors disposed opposite to each other; the two two-dimensional force sensors of the first group are configured to detect respective forces applied to the handle in the first direction and the second direction; the two two-dimensional force sensors of the second group is configured to detect respective forces applied to the handle in the first direction and the third direction; and respective planes in which the first direction, the second direction and the third direction lie are not coplanar.

In some embodiments, the at least four two-dimensional force sensors are arranged in a quadrilateral configuration; the quadrilateral is a planar quadrilateral or a three-dimensional quadrilateral.

In some embodiments, a plane defined by the quadrilateral is parallel to a plane defined by the handle; the quadrilateral is a rectangle; the two two-dimensional force sensors of the first group are symmetrically arranged on two sides of the rectangle, and the two two-dimensional force sensors of the second group are symmetrically arranged on other two sides of the rectangle; or, the four two-dimensional force sensors are respectively arranged at a center of each side of the rectangle; or, the two-dimensional force sensors are respectively arranged at each corner of the rectangle.

In some embodiments, the force sensor group comprises at least three three-dimensional force sensors; the three three-dimensional force sensors are arranged at three vertices of a triangle; a plane defined by the triangle is parallel to a plane defined by the handle; the three-dimensional force sensors detects respective forces applied to the handle in the first direction, the second direction and the third direction; and respective planes in which the first direction, the second direction and the third direction lie are not coplanar.

In some embodiments, the first direction, the second direction, and the third direction are mutually perpendicular; and the first direction is perpendicular to a plane defined by the handle.

In some embodiments, the combined multi-dimensional force sensor further comprises a first fixed frame and a second fixed frame; the force sensor group is mounted between the first fixed frame and the second fixed frame; the first fixed frame is connected to the control head; and the second fixed frame is connected to the handle.

In some embodiments, the force sensor group is fixedly connected to the first fixed frame and the second fixed frame.

In some embodiments, the force sensor group is connected to the first fixed frame and the second fixed frame; there exists a movement clearance between the force sensor group and at least one of the first fixed frame and the second fixed frame; and the force sensor group is movable within the clearance.

In some embodiments, a connecting portion between the handle and the control head has a rectangular structure; the rectangular structure is connected to the second fixed frame; and the outer contours of the first fixed frame and the second fixed frame are aligned with the outer contour of the rectangular structure.

In some embodiments, the combined multi-dimensional force sensor comprises a six-dimensional force sensor; the six-dimensional force sensor detects forces applied to the handle in the first direction, the second direction and a third direction, and outputs the detection signals representing movement of the handle along the X-axis direction, movement of the handle along the Y-axis direction, movement of the handle along the Z-axis direction, rotation of the handle about the Z-axis direction, rotation of the handle about the first direction, and/or rotation of the handle about the second direction.

In some embodiments, the second end of the lifting arm is rotatably connected to the rotating arm; or, the second end of the lifting arm is rotatably connected to the first end thereof.

An X-ray generation device is provided in an embodiment, comprising:

a suspension assembly, comprising a first guide rail, a second guide rail, a moving member, a lifting arm, and a rotating arm, wherein the first guide rail extends along an X-axis direction, the second guide rail extends along a Y-axis direction, the second guide rail is movably connected to the first guide rail and is movable relative to the first guide rail along the X-axis direction, the moving member is movably connected to the second guide rail and is movable relative to the second guide rail along the Y-axis direction, the lifting arm has a first end and a second end that are opposite to each other, the first end of the lifting arm is connected to the moving member, and the second end of the lifting arm is liftable relative to the first end along the Z-axis direction; the rotating arm is connected to the second end of the lifting arm and is rotatable relative to the first end of the lifting arm about the Z-axis direction; and

a head assembly, comprising a connection base, a bracket, a control head and a handle, wherein the connection base is connected to the rotating arm, and the bracket is rotatably connected to the connection base and is rotatable relative to the connection base about a first direction, the control head is configured to emit X-rays to a region to be examined, the control head is rotatably connected to the bracket and is rotatable relative to the bracket about a second direction, the handle is connected to the control head and is operable by a user to effectuate the movement, lifting, and/or rotation.

In some embodiments, the X-ray generation device further comprises:

an electric assist assembly, comprising at least one electric assist member, wherein the at least one electric assist member is connected to at least one of the second guide rail, the moving member, the lifting arm, the rotating arm, the bracket, and the control head; the at least one electric assist member is configured to drive at least one of: movement of the second guide rail relative to the first guide rail along the X-axis direction, movement of the moving member relative to the second guide rail along the Y-axis direction, lifting of the second end of the lifting arm relative to the first end along the Z-axis, rotation of the rotating arm relative to the lifting arm about the Z-axis, rotation of the bracket relative to the rotating arm about the first direction, and rotation of the control head relative to the bracket about the second direction;

a combined multi-dimensional force sensor, connected to the control head and the handle, wherein the combined multi-dimensional force sensor is configured to detect a force direction and a force magnitude of the handle, and generate corresponding detection signals; and

a controller, in signal communication with the combined multi-dimensional force sensor and the at least one electric assist member, wherein the controller is configured to obtain the detection signals and generate corresponding control signals, and send the control signals to the at least one electric assist member, so as to control the movement, lifting, and/or rotation of the control head.

In some embodiments, the combined multi-dimensional force sensor comprises a force sensor group; the force sensor group comprises at least four two-dimensional force sensors; the at least four two-dimensional force sensors are divided into a first group and a second group; the first group and the second group each comprises two two-dimensional force sensors disposed opposite to each other; the two two-dimensional force sensors of the first group are configured to detect respective forces applied to the handle in the first direction and the second direction; the two-dimensional force sensors of the second group is configured to detect respective forces applied in the first direction and the third direction;

and respective planes in which the first direction, the second direction and the third direction lie are not coplanar.

In some embodiments, the combined multi-dimensional force sensor further comprises a first fixed frame and a second fixed frame; the force sensor group is mounted between the first fixed frame and the second fixed frame; the first fixed frame is connected to the control head; and the second fixed frame is connected to the handle.

In some embodiments, a connecting portion between the handle and the control head has a rectangular structure; the rectangular structure is connected to the second fixed frame; and the outer contours of the first fixed frame and the second fixed frame are aligned with the outer contour of the rectangular structure.

In some embodiments, the first direction, the second direction, and the third direction are mutually perpendicular; and the first direction is perpendicular to a plane defined by the handle.

An X-ray generation device is provided in an embodiment, comprising:

a suspension assembly, comprising a first guide rail, a second guide rail, a moving member, a lifting arm, and a rotating arm, wherein the first guide rail extends along an X-axis direction, the second guide rail extends along a Y-axis direction, the second guide rail is movably connected to the first guide rail and is movable relative to the first guide rail along the X-axis direction, the moving member is movably connected to the second guide rail and is movable relative to the second guide rail along the Y-axis direction; the lifting arm has a first end and a second end that are opposite to each other, the first end of the lifting arm is connected to the moving member, the second end of the lifting arm is liftable relative to the first end along a Z-axis direction, the rotating arm is connected to the second end of the lifting arm, and the rotating arm is rotatable relative to the second end of the lifting arm about the Z-axis direction;
a head assembly, comprising a connection base, a bracket, a control head, and a handle, wherein the connection base is connected to the rotating arm, the bracket is rotatably connected to the connection base, the bracket is rotatable relative to the connection base about the first direction, the control head is configured to emit X-rays to a region to be examined and is connected to the bracket; and the handle is connected to the control head and is operable by a user to effectuate the movement, lifting, and/or rotation of the control head;
an electric assist assembly, comprising at least one electric assist member, wherein the at least one electric assist member is connected to at least one of the second guide rail, the moving member, the lifting arm, the rotating arm, and the bracket; the at least one electric assist member is configured to drive: movement of the second guide rail relative to the first guide rail along the X-axis, movement of the moving member relative to the second guide rail along the Y-axis direction, lifting of the second end of the lifting arm relative to the first end along the Z-axis direction, rotation of the rotating arm relative to the lifting arm about the Z-axis, and rotation of the bracket relative to the rotating arm about the Y-axis direction;
a combined multi-dimensional force sensor, connected to the control head and the handle combined multi-dimensional force sensor, wherein the combined multi-dimensional force sensor detects a force direction and a force magnitude of the handle and generates corresponding detection signals; and
a controller, in signal communication with the combined multi-dimensional force sensor and the at least one electric assist member, wherein the controller obtains the detection signals, generates corresponding control signals, and sends the control signals to the at least one electric assist member, so as to control the movement, lifting, and/or rotation of the control head.
the electric assist assembly comprises a first electric assist member, a second electric assist member, a third electric assist member, a fourth electric assist member, and fifth electric assist member; the first electric assist member is connected to the second guide rail so as to drive the second guide rail to move along the X-axis direction; the second electric assist member is connected to the moving member so as to drive the moving member to move along the Y-axis direction; the third electric assist member is connected to the lifting arm so as to drive the second end of the lifting arm to lift relative to the first end along the Z-axis; the fourth electric assist member is connected to the rotating arm so as to drive the rotating arm to rotate relative to the lifting arm about the Z-axis; and the fifth electric assist member is connected to the bracket so as to drive the bracket to rotate relative to the rotating arm about the first direction;
the controller, in signal communication with the first electric assist member, the second electric assist member, third electric assist member, the fourth electric assist member, and the fifth electric assist member, wherein the controller obtains the detection signals, generates corresponding control signals, and sends the control signals to one or more of the first electric assist member, the second electric assist member, the third electric assist member, the fourth electric assist member, and the fifth electric assist member, so as to control the movement, lifting, and/or rotation of the control head.

In some embodiments, the combined multi-dimensional force sensor comprises a force sensor group; the force sensor group comprises at least four two-dimensional force sensors; the at least four two-dimensional force sensors are divided into a first group and a second group; the first group and the second group each comprises two two-dimensional force sensor disposed opposite to each other; the two-dimensional force sensors of the first group detect respective forces applied to the handle in the first direction and the second direction; and the two two-dimensional force sensors of the

second group detect the forces applied to the handle in the first direction and the third direction.

In some embodiments, the combined multi-dimensional force sensor further comprises a first fixed frame and a second fixed frame; the force sensor group is mounted between the first fixed frame and the second fixed frame; the first fixed frame is connected to the control head; and the second fixed frame is connected to the handle.

In some embodiments, a connecting portion between the handle and the control head has a rectangular structure; the rectangular structure is connected to the second fixed frame; and the outer contours of the first fixed frame and the second fixed frame are aligned with the outer contour of the rectangular structure.

An X-ray imaging system is provided in an embodiment, comprising:

the X-ray generation device mentioned above; and
a flat panel detector, configured to be placed at a first imaging position detached from a cassette that houses the flat panel detector, and further configured to be placed at a second imaging position housed within the cassette; wherein the control head aligns the flat panel detector; the flat panel detector collects X-rays penetrating the region to be examined and generates corresponding imaging signals that are used for obtaining a captured X-ray image.

According to the X-ray generation device and X-ray imaging system disclosed in the above embodiments, since the handle can drive the control head to move along the X-axis direction, move along the Y-axis direction, lift along the Z-axis direction, rotate about the Z-axis, rotate about the first direction, and rotate about the second direction, the control head has six degrees of freedom. Moreover, the coordinate system in which the first direction and the second direction are defined rotates along with the rotation about the Z-axis, such that the control head is capable of moving arbitrarily within a total of six degrees of freedom across the two coordinate systems. The control head can be moved to any position within this range to perform imaging on a patient, thereby meeting imaging requirements for patients in different postures and at different body regions. Furthermore, the X-ray generation device is provided with six electric assist members. The six electric assist members drives the movement of the control head in the six degrees of freedom, such that medical personnel only need to apply a small force to the handle to drive the movement of the control head, thereby saving time and effort.

## BRIEF DESCRIPTION OF THE DRAWINGS

FIG. 1 is a structural schematic diagram of an X-ray generation device according to one embodiment;
FIG. 2 is a structural schematic diagram of an X-ray generation device according to one embodiment;
FIG. 3 is a structural schematic diagram of a head assembly according to one embodiment;
FIG. 4 is a structural schematic diagram of a head assembly according to one embodiment;
FIG. 5 is a structural block diagram of a control part of an X-ray generation device according to one embodiment;
FIG. 6 is a partial structural schematic diagram of a suspension assembly according to one embodiment;
FIG. 7 is a structural schematic diagram of a first electric assist member according to one embodiment;
FIG. 8 is a structural schematic diagram of a second electric assist member according to one embodiment;
FIG. 9 is a structural schematic diagram of a third electric assist member according to one embodiment;
FIG. 10 is a structural schematic diagram of an X-ray generation device according to one embodiment;
FIG. 11 is a structural schematic diagram of a fourth electric assist member according to one embodiment;
FIG. 12 is a structural schematic diagram of a connection structure between the rotating arm and the lifting arm according to one embodiment;
FIG. 13 is a structural schematic diagram of a first limiting structure and a positioning structure according to one embodiment;
FIG. 14 is a structural schematic diagram of a fifth electric assist member according to one embodiment;
FIG. 15 is a structural schematic diagram of a head assembly according to one embodiment;
FIG. 16 is a structural schematic diagram of a third limiting structure according to one embodiment;
FIG. 17 is a structural schematic diagram of a third limiting structure according to one embodiment;
FIG. 18 is a schematic distribution diagram of four two-dimensional force sensors according to one embodiment;
FIG. 19 is an exploded structural schematic diagram of a combined multi-dimensional force sensor according to one embodiment;
FIG. 20 is a distribution diagram of three three-dimensional force sensors according to one embodiment;
FIG. 21 is a structural block diagram of a control part of an X-ray generation device according to one embodiment; and
FIG. 22 is a structural block diagram of a control part of an X-ray generation device according to one embodiment.

Reference numerals:

10, suspension assembly; 11, guide rail; 111, first guide rail; 112, second guide rail; 12, moving member; 13, lifting arm; 131, first lifting sub-arm; 132, second lifting sub-arm; 133, first rotating shaft; 14, rotating arm; 15, first limiting structure; 151, first limiting member; 1511, swing member; 15111, first arc-shaped hole; 1512, angle limiting member; 152, second limiting member; 16, positioning structure; 161, first positioning member; 1611, positioning hole; 162, second positioning member; 1621, elastic part;

20, head assembly; 21, connection base; 22, bracket; 221, second rotating shaft; 23, control head; 231, operating interface; 24, handle; 25, second limiting structure; 251, third limiting member; 252, fourth limiting member; 26, third limiting structure; 261, fifth limiting member; 2611, second arc-shaped groove; 262, sixth limiting member;

30, electric assist assembly; 31, first electric assist member; 311, first drive motor; 312, first drive pulley; 313, first drive belt; 32, second electric assist member; 321, second drive motor; 322, second drive pulley; 323, second drive belt; 33, third electric assist member; 331, third drive motor; 332, first transmission assembly; 3321, third drive pulley; 3322, third drive belt; 3323, roller shaft; 333, traction element; 34, fourth electric assist member; 341, fourth drive motor; 242, first helical gear; 343, second helical gear; 35, fifth electric assist member; 351, fifth drive motor; 352, transmission shaft; 36, sixth electric assist member; 361, sixth drive motor; 362, second transmission assembly; 3621, fourth drive pulley; 3622, fourth drive belt;

40, combined multi-dimensional force sensor; 41, two-dimensional force sensor; A1, first two-dimensional force sensor; A2, second two-dimensional force sensor; B1, third two-dimensional force sensor; B2, fourth two-dimensional force sensor; 42, first fixed frame; 43, second fixed frame; 44, three-dimensional force sensor;

50, control.

## DETAILED DESCRIPTION

Specific embodiments of the present disclosure will now be described in detail with reference to the accompanying drawings. Similar or related components in different embodiments are labeled with associated reference numerals. The following embodiments include detailed descriptions to facilitate understanding of the present disclosure. However, those skilled in the art will readily recognize that certain features may be omitted under specific circumstances or substituted by other components, materials, or methods. In some instances, certain operations related to the present disclosure are not explicitly described or illustrated herein. This intentional exclusion is intentional to avoid obscuring the core technical solutions of the present disclosure. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

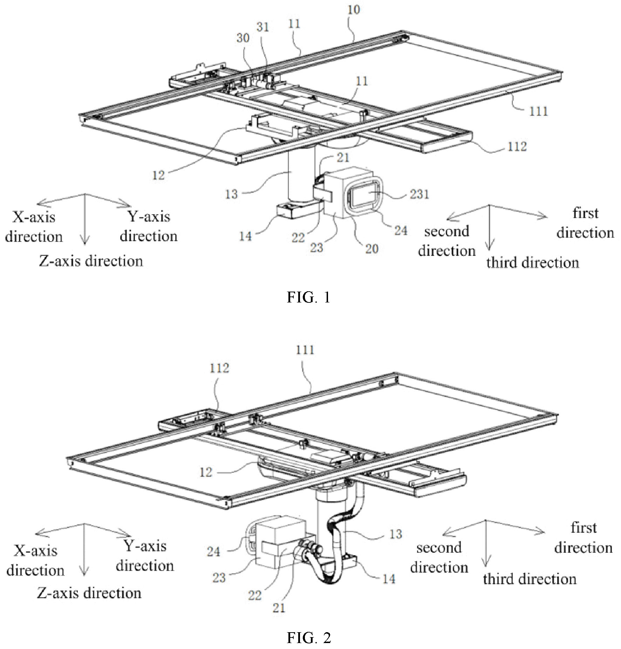

The numerical designations assigned to components herein, such as "first", "second", and the like, are used solely to distinguish between the described objects and carry no implication of order or technical significance. The terms "connected" and "coupled" as used in this application, unless otherwise specified, encompass both direct and indirect connections. Herein, the X-axis, Y-axis and Z-axis represent three mutually perpendicular directional axes, while the first direction, the second direction and the third direction represent three mutually perpendicular directions. The X-axis and Y-axis are two horizontal directional axes, and the Z-axis is a vertical directional axis. In the state shown in FIG. 1, the first direction is parallel to the Y-axis, the second direction is parallel to the X-axis, and the third direction is parallel to the Z-axis. The coordinate system formed by the first, second, and third directions changes with rotation about the Z-axis. That is, the X-axis, Y-axis and Z-axis represent fixed directions relative to the ceiling, whereas the coordinate system in which the first, second, and third directions are are defined rotates with the rotation about the Z-axis. The mutual perpendicularity of the X-axis, Y-axis, and Z-axis, as well as the mutual perpendicularity of the first, second, and third directions, encompasses both strict perpendicularity and approximate perpendicularity. In cases of approximate perpendicularity, the corresponding movement or rotation can be achieved through algorithm compensation.

In some embodiments, an X-ray generation device is provided. The X-ray generation device is suspended from the ceiling and generates and emits X-rays. The X-ray generation device operates in conjunction with a flat panel detector. The X-ray generation device emits X-rays to a region of a patient to be examined, and the flat panel detector collects X-rays that have penetrated the patient and generate corresponding imaging signals. These imaging signals are processed to obtain a captured X-ray image.

The X-ray generation device in the present embodiment is a multi-degree-of-freedom apparatus. The control head has three mutually perpendicular linear degrees of freedom (movement) and three mutually perpendicular rotational degrees of freedom relative to the ceiling. Within its range of motion, the control head can be positioned at any point through a combination of translation, lifting, and rotation, thereby expanding the application scenarios of the X-ray generation device. For example, by utilizing its six degrees of freedom, the control head can be moved to align with the region to be

examined of patients in various postures, such as standing, lying down, or sitting. This capability is particularly advantageous for facilitating imaging of patients with limited mobility.

The X-ray generation device in the present embodiment is further provided with an electric assist function. The X-ray generation device comprises a force sensor and an electric assist member. When a medical operator manipulates the handle, the force sensor detects the force applied to the handle, calculates and analyzes it to determine the magnitude and direction of the operator's input force. The electric assist member then drives the control head and the handle to move in accordance with the operator's manipulation. This allows the medical operator to effortlessly maneuver the control head with minimal force, and even enables single-handed operation for the operator. Concurrently, this functionality enhances the precision with which the operator can position the control head via the handle, ensuring accurate alignment with the region to be examined of the patient and thereby improving imaging efficiency.

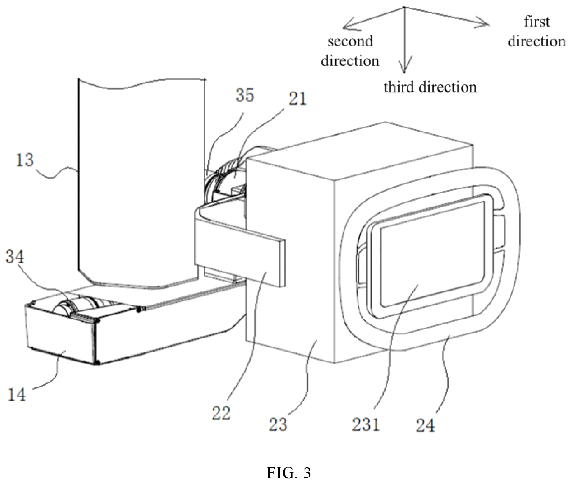

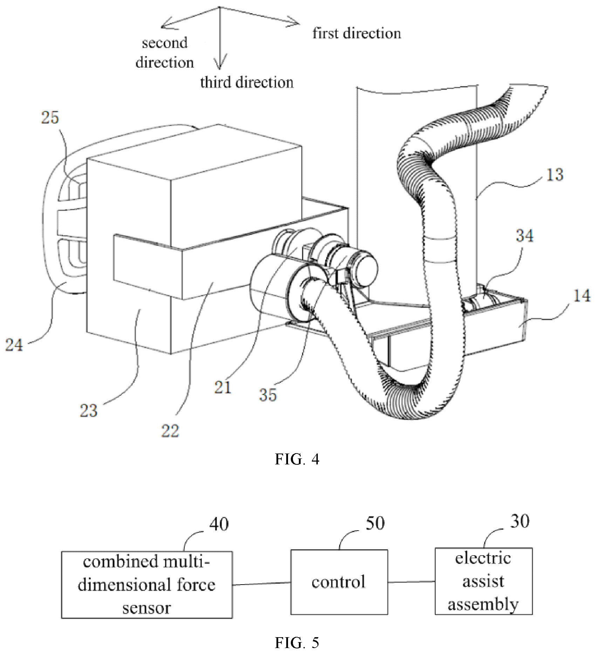

Referring to FIGS. 1 to 5, the X-ray generation device in the present embodiment primarily comprises a suspension assembly 10, a head assembly 20, an electric assist assembly 30, a combined multi-dimensional force sensor 40, and a controller 50. The suspension assembly 10 is connected to the ceiling, the head assembly 20 is mounted to the lower end of the suspension assembly 10. The electric assist assembly 30 is disposed in the suspension assembly 10 and the head assembly 20. The combined multi-dimensional force sensor 40 is arranged in the electric assist assembly 30. The controller 50 may be arranged inside the head assembly 20 or in an external host device, and communicates with the head assembly 20, the electric assist assembly 30, and the combined multi-dimensional force sensor 40 via wired or wireless communication.

The suspension assembly 10 functions as a supporting structure to suspend the head assembly 20 in an examination room. The head assembly 20 is configured with multiple degrees of freedom, enabling it to be moved horizontally, vertically (lifting), and rotated.

The suspension assembly 10 primarily comprises a guide rail 11, a moving member 12, a lifting arm 13, and a rotating arm 14. The guide rail 11 includes a first guide rail 111 and a second guide rail 112. The first guide rail 111 extends and fixes along the X-axis direction on the ceiling. The second guide rail 112 extends along the Y-axis direction on the first guide rail 111 and is movable relative to the first guide rail 111 along the X-axis direction. The first guide rail 111 may comprise two parallel rails, forming a frame-like structure. Similarly, the second guide rail 112 may comprise two parallel rails, forming a frame-like structure. The use of a dual-track, frame-type structure for both the first guide rail 111 and the second guide rail 112 enhances force distribution uniformity and improves motion stability.

The moving member 12 is movably connected to the guide rail 11, and the moving member 12 is movably connected to the second guide rail 112. The moving member 12 moves along the X-axis direction together with the second guide rail 112 relative to the first guide rail 111, and further moves relative to the second guide rail 112 along the Y-axis direction. Herein, the X-axis direction and the Y-axis direction are horizontal directions. Since the X-ray generation device in this embodiment has six degrees of freedom, the X-axis direction and the Y-axis direction may also be inclined relative to the horizontal plane. For example, when the ceiling is sloped, the X-ray generation device can be mounted on the inclined ceiling and remains capable of maneuvering the control head to any desired position for imaging.

The moving member 12 may be implemented as a moving trolley or a sliding block. The moving member 12 drives the entire X-ray emission device to move along the X-axis direction and the Y-axis direction.

The lifting arm 13 comprises a first end and a second end that are opposite to each other. The first end of the lifting arm 13 may be the upper end, and the second end of the lifting arm 13 may be the lower end. The first end of the lifting arm 13 is connected to the moving member 12, and the lifting arm 13 is suspended from the lower end of the moving member 12. The second end of the lifting arm 13 is liftable relative to the first end along the Z-axis direction, so as to drive loads such as the head assembly 20 to move vertically along the Z-axis direction.

The lifting arm 13 may comprise at least two lifting columns. The at least two lifting columns are movably connected in sequence along the Z-axis direction, forming at least two telescopic sections capable of vertical movement. For example, the lifting arm 13 includes three lifting columns connected sequentially from top to bottom, with the outer and inner diameters of the three lifting columns decreasing progressively from top to bottom. The upper end of the uppermost lifting column serves as the first end of the lifting arm 13, and the lower end of the lowermost lifting column serves as the second end of the lifting arm 13. The three lifting columns are configured as hollow structures to facilitate retraction, extension, and internal cable routing. When fully lifted to the highest position, the three lifting columns are telescopically nested within one another, with the lower two columns concealed within the uppermost column. When fully lowered to the lowest position, the lower two lifting columns are extended and exposed.

The rotating arm 14 is connected to the second end of the lifting arm 13 so as to rotate relative to the second end of the lifting arm 13 about the Z-axis. The rotating arm 14 and the second end of the lifting arm 13 are swingably connected via components such as a rotating shaft and a bearing. That is, a rotational joint is provided at the lowermost end of the lifting arm 13, minimizing the moment of inertia about the Z-axis and thereby improving the stability and accuracy of the rotational movement about the Z-axis. The rotating arm 14 may be perpendicularly connected to the lifting arm 13, meaning the rotating arm 14 is horizontally disposed and rotates about the Z-axis in the horizontal plane.

In other embodiments, the rotational joint of the lifting arm 13 may also be provided at an upper end or middle

position. The rotating arm 14 is fixedly connected to the second end of the lifting arm 13, and the second end of the lifting arm 13 is rotatably connected to the first end of the lifting arm 13. That is, the second end of the lifting arm 13 can lift along the Z-axis direction and rotate relative to the first end about the Z-axis. For example, among the three lifting columns of the lifting arm 13, the uppermost lifting column may be rotatably connected to the middle lifting column, or the middle lifting column may be rotatably connected to the lowermost lifting column. The lowermost lifting column is fixedly connected to the rotating arm 14, enabling the lower lifting column(s) to drive the rotating arm 14 to rotate about the Z-axis direction.

In this embodiment, the head assembly 20 primarily comprises a connection base 21, a bracket 22, a control head 23, and a handle 24. The connection base 21 is connected to the rotating arm 14. The connection base 21 may be an integral structure with the rotating arm 14, or may be fixedly connected to the rotating arm 14 by means such as screws, snap-fit engagement, or welding.

The bracket 22 is rotatably connected to the connection base 21 and can rotate relative to the connection base 21 along the first direction. The first direction is perpendicular to the longitudinal direction of the connection base 21. In the position state shown in FIG. 1, the first direction is parallel to the Y-axis direction. When the control head 23 is rotated about the Z-axis to other positions, the first direction will intersect with the Y-axis direction. The bracket 22 may be rotatably connected via components such as a rotating shaft and a bearing. The connection base 21 may be provided with a mounting hole, wherein one end of the rotating shaft is rotatably connected to the connection base 21, and the other end of the rotating shaft is fixedly connected to the bracket 22. Providing the rotational joint on the connection base 21 simplifies the structure of the bracket 22, thereby facilitating the bracket 22 to free up more space for the rotation of the control head 23.

The control head 23 generates X-rays and emits them to a patient's region to be examined. The control head 23 is rotatably connected to the bracket 22 and can rotate relative to the bracket 22 about the second direction. In the position state shown in FIG. 1, the second direction is parallel to the X-axis direction. When the control head 23 rotates about the Z-axis to other positions, the second direction will intersect with the Z-axis direction.

The bracket 22 may be of a C-shaped structure, with the outer side of the middle part of the bracket 22 rotatably connected to the connection base 21. The two ends of the control head 23 are respectively rotatably connected to the two ends of the bracket 22 via components such as a rotating shaft and a bearing. The control head 23 is disposed inside the C-shaped structure of the bracket 22. The bracket 22 can drive the control head 23 to rotate together along the first direction.

In other embodiments, the bracket 22 may also have a straight rod structure. A C-shaped structure is provided on one side of the control head 23, and the straight rod structure of the bracket 22 is rotatably connected within the C-shaped structure of the control head 23. This configuration also enables a rotatable connection between the control head 23 and the bracket 22, and allows the control head 23 to rotate relative to the bracket 22 about the second direction.

In the present embodiment, the handle 24 is connected to the control head 23. One side of the control head 23 faces the bracket 22 and the connection base 21, while the other side faces the handle 24. That is, the handle 24 and the bracket 22 are arranged on opposite sides of the control head 23, so that the bracket 22 does not interfere with the use of the handle 24.

The handle 24 and the control head 23 may be fixedly connected. The handle 24 can drive the control head 23 to perform movements including: translation along the X-axis direction, translation along the Y-axis direction, translation along the Z-axis direction, rotation about the Z-axis direction, rotation about the first direction, and rotation about the second direction. The handle 24 and the control head 23 move, translate, and rotate together, with no relative motion occurring between the handle 24 and the control head 23.

The handle 24 comprises a main body portion and a connecting portion. The main body portion of the handle 24 may be configured as a substantially quadrilateral ring structure with unequal lengths of its sides. This asymmetrical design allows medical personnel to tactilely perceive the rotational angle. For example, when the handle 24 is rotated by 90°, its long side moves to the position originally occupied by the shorter side, and vice versa. The connecting portion of the handle 24 is coupled to the control head 23.

An operating interface 231 may be mounted on the outer surface of the connecting portion of the handle 24. The operating interface 231 may be a touch screen or a combination of a display screen and physical buttons. The operating interface 231 is communicatively coupled to the control head 23 and is used to input commands for generating and emitting X-rays.

In this embodiment, the control head 23 has a defined range of motion in its six degrees of freedom. The three-dimensional movement space formed by the translation of the control head 23 along the X-axis, Y-axis, and Z-axis direction covers the standing imaging position, supine imaging position, and other baseed imaging positions. The control head 23 is capable of rotating relative to the bracket 22 about the second direction within an angular range of -10° to +90°; and/or, the bracket 22 is capable of rotating relative to the rotating arm 14 about the first direction within an angular range of -140° to +140°; and/or, the rotating arm 14 is capable of rotating relative to the first end of the lifting arm 13 about the Z-axis direction within an angular range of -180° to +180°. The control head 23 can be oriented toward different examination regions of the patient in standing, supine, or other baseed imaging positions.

The range of motion for the six degrees of freedom of the control head 23 may be configured based on operational

requirements and application scenarios.

In this embodiment, the electric assist assembly 30 provides driving force for the translation and rotation of the control head 23, thereby providing assistance in all six degrees of freedom of the control head 23 and reducing the operational effort required by medical personnel.

The electric assist assembly 30 comprises a first electric assist member 31, a second electric assist member 32, a third electric assist member 33, a fourth electric assist member 34, a fifth electric assist member 35, and a sixth electric assist member 36. These six electric assist members provide powered assistance and drive for the six degrees of freedom of the control head 23, with each electric assist member corresponding to and driving one degree of freedom of the control head 23. Using one electric assist member per degree of freedom helps simplify the structure of the electric assist members, particularly by streamlining the transmission mechanism, and also simplifies the control and drive for each individual degree of freedom. Each of the first electric assist member 31, the second electric assist member 32, the third electric assist member 33, the fourth electric assist member 34, the fifth electric assist member 35, and the sixth electric assist member 36 includes a drive motor, which may direct provide motive power or operate through a transmission mechanism. For example, when driving the translation and vertical movement of the control head 23, the motor may actuate these motions via transmission mechanisms such as worm gears or timing belts and pulleys. For rotational movement of the control head 23, the motor may drive the rotation of the control head 23 directly.

The first electric assist member 31 and the second electric assist member 32 are connected to the moving member 12, respectively. The first electric assist member 31 may be mounted on the ceiling or the second guide rail 112. The first electric assist member 31 is connected to the second guide rail 112 and is indirectly coupled to the moving member 12. The first electric assist member 31 drives the second guide rail 112 and the moving member 12 to move relative to the first guide rail 111 along the X-axis direction. The second electric assist member 32 is mounted on either the second guide rail 112 or the moving member 12 and is directly connected to the moving member 12. The second electric assist member 32 is used to drive the moving member 12 to move relative to the second guide rail 112 along the Y-axis direction.

In other embodiments, the first electric assist member 31 and the second electric assist member 32 may be integrated into a single electric drive unit. The integrated electric drive unit comprises one power source and two sets of transmission mechanisms. The power source is connected to the second guide rail 112 via one transmission mechanism to drive movement along the X-axis direction, and is connected to the moving member 12 via the other transmission mechanism to drive movement along the Y-axis direction. The two transmission mechanism can operate independently and/or concurrently, enabling independent movement along the X-axis direction, independent movement along the Y-axis direction, or simultaneous movement along both the X-axis direction and the Y-axis direction.

In this embodiment, the third electric assist member 33 is mounted on the moving member 12. The third electric assist member 33 is connected to the second end of the lifting arm 13 via a transmission component such as a cable or rope. The third electric assist member 33 drives the second end of the lifting arm 13 to move vertically along the Z-axis direction relative to the first end. That is, the third electric assist member 33 drives the lowermost lifting cylinder of the lifting arm 13 to extend and retract relative to the uppermost lifting cylinder along the Z-axis direction.

In other embodiments, the third electric assist member 33 may also be disposed inside the first end of the lifting arm 13. It is connected to the second end of the lifting arm 13 and may also drive the second end of the lifting arm 13 to move vertically relative to the first end along the Z-axis direction.

The fourth electric assist member 34 is disposed on the second end of the lifting arm 13 or the rotating arm 14. It is connected to the rotating arm 14 and is used to drive the rotating arm 14 to rotate relative to the second end of the lifting arm 13 about the Z-axis direction.

In other embodiments, if the rotational joint about the Z-axis is disposed inside the lifting arm 13, the fourth electric assist member 34 is disposed inside the lifting arm 13, and the fourth electric drive member 34 is used to drive the second end of the lifting arm 13 to rotate relative to the first end about the Z-axis.

In this embodiment, the fifth electric drive member 35 is disposed on the connection base 21 or the bracket 22, and the fifth electric drive member 35 is connected to the bracket 22. The fifth electric drive member 34 is used to drive the bracket 22 to rotate relative to the connection base 21 about the first direction.

The sixth electric drive member 36 is disposed on the bracket 22 or the control head 23, and the sixth electric drive member 36 is connected to the control head 23. The sixth electric drive member 36 is used to drive the control head 23 to rotate relative to the bracket 22 about the second direction.

In this embodiment, the combined multi-dimensional force sensor 40 is connected to the handle 24. The force applied to the handle 24 may be transmitted to the combined multi-dimensional force sensor 40, enabling the sensor 40 to detect the force condition on the handle 24 and generate corresponding detection signals. The combined multi-dimensional force sensor 40 may be disposed between the connecting portion between the control head 23 and the handle 24. The control head 23 provides support for the combined multi-dimensional force sensor 40, thereby allowing the sensor 40 to collect the force on the handle 24.

The combined multi-dimensional force sensor 40, serving as a force sensor, is used to detect the forces applied to the handle 24 in the first, second, and third directions, and output detection signals representing the movement of the

handle 24 along the X-axis, the Y-axis, and the Z-axis, as well as its rotation about the Z-axis, the first direction, and/or the second direction. In other words, the combined multi-dimensional force sensor 40 can derive and calculate the degrees of freedom and the magnitude of the electric assistance required for the control head 23 by detecting the forces applied to the handle 24 in the first, second, and third directions, and then combining the posture of the handle 24.

The controller 50 may be disposed on the control head 23 and is in signal communication with the combined multi-dimensional force sensor 40, as well as the first through sixth electric assist members 31, 32, 33, 34, 35, and 36. The controller 50 obtains the detection signals generated by the combined multi-dimensional force sensor 40, calculates corresponding control signals based on the detection signals, and transmits the control signals to one or more of the first through sixth electric assist members 31-36, so as to control and drive the movement, lifting, and/or rotation of the control head 23. Specifically, when the controller 50 determines from the detection signals that the motion of the control head 23 along only one degree of freedom needs to be controlled, it sends the control signals to one of the electric assist members 31-36 to control and drive the movement, lifting, or rotation of the control head 23. If the controller 50 calculates based on the detection signals that the motion of the control head 23 along at least two degrees of freedom is required, it sends the control signals to two or more of the electric assist members 31-36 to control one or more of the movement, lifting, and rotation of the control head 23. Furthermore, the controller 50 can simultaneously control the multiple electric assist members to achieve fitted motion of the control head 23. For example, it can enable simultaneous movement and rotation of the control head 23 to fit the three-dimensional trajectory of its movement as guided by medical personnel.

In other embodiments, the controller 50 may also be disposed in an external host device, and may be connected to the control head 23 wiredly or wirelessly. The controller 50 is in signal communication with the combined multi-dimensional force sensor 40, as well as the first through sixth electric assist members 31, 32, 33, 34, 35, and 36 via the control head 23.

In this embodiment, the handle 24 enables the control head 23 to achieve movement along the X-axis, movement along the Y-axis, lifting along the Z-axis, rotation about the Z-axis, rotation about the first direction, and rotation about the second direction. Thus, the control head 23 possesses six degrees of freedom. Moreover, the coordinate system in which the first direction and the second direction reside will rotate along with the Z-axis when the Z-axis rotates. This allows the control head 23 to move freely within a total of six degrees of freedom across these two coordinate systems, resulting in high flexibility. The control head 23 can be positioned at any point within this range to perform imaging on patients, thereby accommodating different patient postures and various anatomical regions. Furthermore, the X-ray generation device is equipped with six electric assist members that can drive the movement of the control head 23 across its six degrees of freedom. This design allows medical personnel to maneuver the control head 23 by applying only a minimal force to the handle 24, which saves both time and effort.

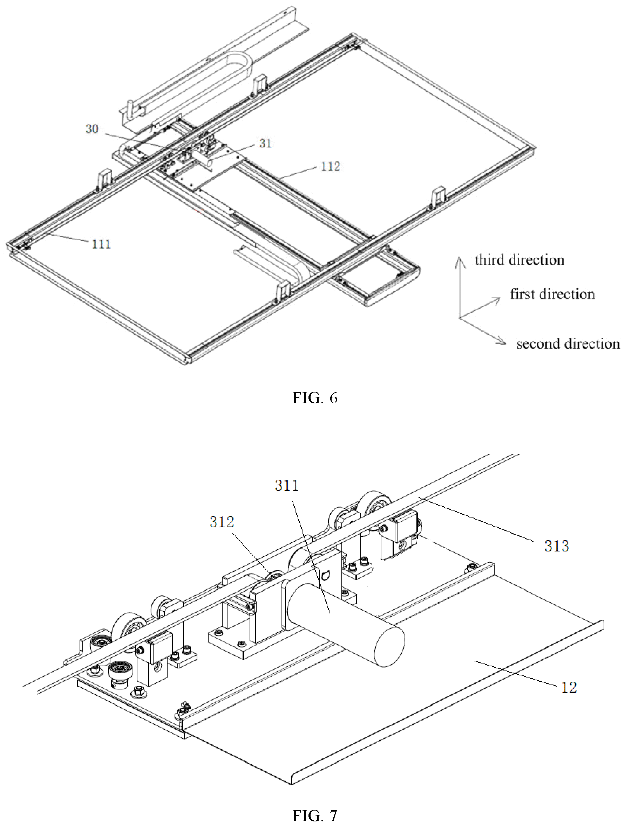

Referring to FIGS. 6 and 7, in some embodiments, the first electric assist member 31 includes a first drive motor 311, a first drive pulley 312, and a first drive belt 313. The first guide rail 111 may be directly fixed to the ceiling, and the second guide rail 112 may be installed under the first guide rail 111 so as to be movable along the X-axis. The first drive motor 311 and the first drive pulley 312 are installed on the second guide rail 112, and the first drive pulley 312 may be directly installed on the output shaft of the first drive motor 311. The first drive belt 313 is installed on the first guide rail 111 along the X-axis and can be a fixed structure (e.g., non-movable relative to the first guide rail). The output shaft of the first drive motor 311 is fixedly connected to the first drive pulley 312, and the first drive pulley 312 is in transmission connection with the first drive belt 313. The first drive pulley 312 may roll along the length of the first drive belt 313. The first drive motor 311 is used to drive the first drive pulley 312 to move relative to the first drive belt 313 along the X-axis, thereby driving the second guide rail 112 to move relative to the first guide rail 111 along the X-axis.

The first drive motor 311 is in signal communication with the controller 50, and the controller 50 can control the first drive motor 311 to drive the second guide rail 112 to move relative to the first guide rail 111 along the X-axis to an accurate target position, thereby driving the control head 23 to move along the same axis to a predetermined position.

In other embodiments, the first drive motor 311 may also be mounted on the first guide rail 111, and the first drive belt 313 may be drivably mounted on the first guide rail 111 via the first drive pulley 312. The second guide rail 112 may be fixedly connected to the first drive belt 313 by a connecting block. By actuating the first drive belt 313 to move along the X-axis, the first drive motor 311 may thereby drive the second guide rail 112 to move relative to the first guide rail 111 along the X-axis.

In other embodiments, the first electric assist member 31 may also be implemented as an alternative drive mechanism. For example, the first electric assist member 31 may comprises a drive motor, a sprocket, and a drive chain. The drive motor may also drive the second guide rail 112 to move relative to the first guide rail 111 along the X-axis by means of the sprocket and the drive chain.

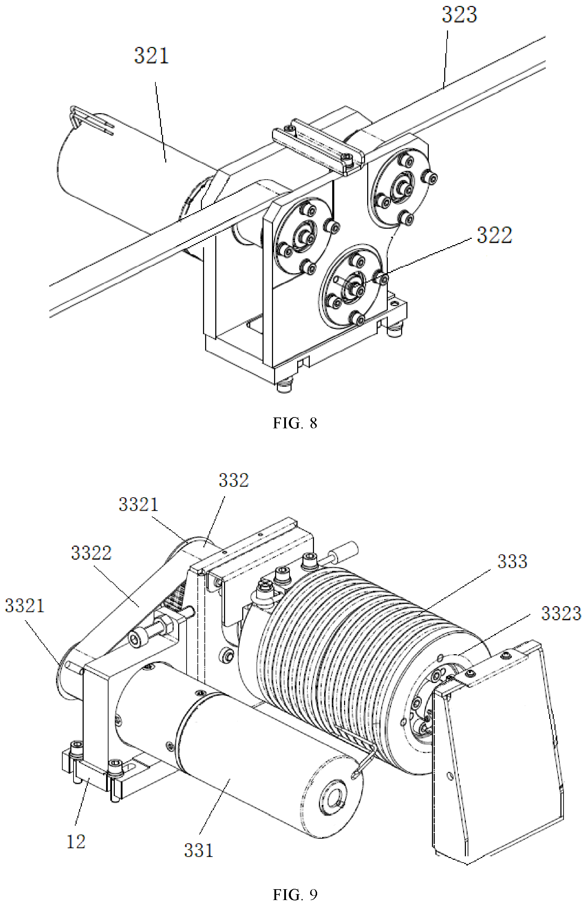

As shown in FIG. 8, in an embodiment, the second electric assist member 32 includes a second drive motor 321, a second drive pulley 322 and a second drive belt 323. The second drive motor 321 and the second drive pulley 322 are fixedly mounted on the moving member 12. The second drive belt 323 is mounted on the second guide rail 112 along the Y-axis direction. The second drive belt 323 may be a fixed structure. The output shaft of the second drive motor 321 is fixedly connected to the second drive pulley 322, and the second drive pulley 322 is in transmission connection with the second

drive belt 323. The second drive pulley 322 may roll along the length of the second drive belt 323. The second drive motor 321 is used to drive the second drive pulley 322 to move relative to the second drive belt 323 along the Y-axis direction, thereby driving the moving member 12 to move relative to the second guide rail 112 along the Y-axis direction.

The second electric assist member 32 is signally connected to the controller 50. The controller 50 can control the second electric assist member 32 to drive the moving member 12 to move relative to the second guide rail 112 along the Y-axis direction to a precise target position, thereby moving the control head 23 to a predetermined position along the Y-axis direction.

In other embodiments, the second drive motor 321 may also be mounted on the second guide rail 112. The second drive belt 323 is drivably mounted on the second guide rail 112 via the second drive pulley 322. The moving member 12 is fixedly connected to the second drive belt 323 by a connecting block. By driving the second drive belt 323 to move along the Y-axis direction, the second drive motor 321 can also drive the moving member 12 to move relative to the second guide rail 112 along the Y-axis direction.

In other embodiments, the second electric assist member 32 may also be an alternative drive mechanism. For example, the second electric assist member 32 may include a drive motor, a sprocket and a drive chain. The drive motor can also drive the moving member 12 to move, via the sprocket and the drive chain, relative to the second guide rail 112 along the Y-axis direction.

As shown in FIG. 9, in an embodiment, the third electric assist member 33 includes a third drive motor 331, a first transmission assembly 332, and a traction element 333. The third drive motor 331, the first transmission assembly 332, and the traction element 333 are mounted on the moving member 12. The third drive motor 331 is fixedly to the moving member 12 and is connected to the traction element 333 via the first transmission assembly 332. The traction element 333 extends to and is connected to the second end of the lifting arm 13. The third drive motor 331 drives the traction element 333 to move vertically via the first transmission assembly 332, thereby causing the second end of the lifting arm 13 to move vertically along the Z-axis direction and thus driving the control head 23 to move vertically along the Z-axis direction.

The first transmission assembly 332 includes two third drive pulleys 3321, a third drive belt 3322, and a roller shaft 3323. The output shaft of the third drive motor 331 is fixedly connected to one of the third drive pulleys 3321. The roller shaft 3323 is rotatably mounted on the moving member 12 and is fixedly connected to the other of the third drive pulleys 3321. The two third drive pulleys 3321 are drivably connected by the third drive belt 3322. The traction element 333 is a flexible traction structure, such as a traction cable. The traction cable is wound around the roller shaft 3323, with one end fixedly connected to the roller shaft 3323 and the other end secured to the second end of the lifting arm 13. Rotation of the roller shaft 3323 causes the other end of the traction cable to move vertically, thereby driving the second end of the lifting arm 13 to lift relative to its first end.

The roller shaft 3323 has a specific diameter, thereby providing a mechanical advantage (moment arm) that enables it to drive the second end of the lifting arm 13 to move vertically relative to the first end more effectively. Additionally, the diameter provides a larger winding surface for accommodating the traction cable.

By configuring the traction element 333 as a flexible traction cable, the vertical travel range along the Z-axis can be increased. Moreover, the traction cable can be wound for storage, thereby reducing space requirements.

In other embodiments, the first transmission assembly 332 may employ alternative transmission mechanisms. For example, the first transmission assembly 332 may comprise a transmission gear set and a roller shaft. The third drive motor 331 can also drive the roller shaft to rotate via the transmission gear set, thereby causing the second end of the lifting arm 13 to move vertically relative to the first end.

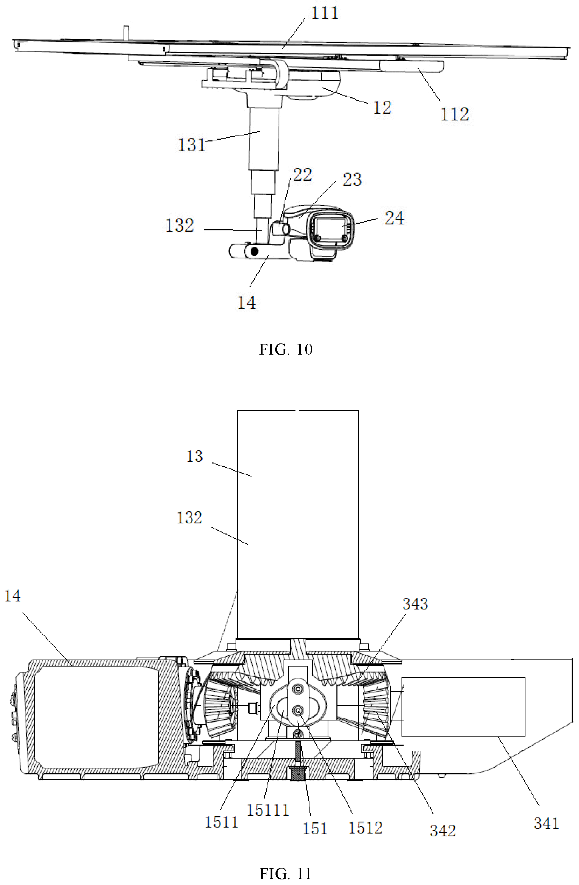

As shown in FIG. 10, in an embodiment, the lifting arm 13 includes at least two lifting arms. Alternatively, the lifting arm 13 may include three or four lifting arms connected in series, wherein adjacent lifting arms move vertically relative to each other. By way of example, the following description is based on an embodiment in which the lifting arm 13 includes a first lifting sub-arm 131 and a second lifting sub-arm 132.

The first lifting sub-arm 131 and the second lifting sub-arm 132 are movably connected for vertical movement along the Z-axis direction. The end of the first lifting sub-arm 131 distal to the second lifting sub-arm 132 serves as the first end of the lifting arm 13, while the end of the second lifting sub-arm 132 distal to the first lifting sub-arm 131 serves as the second end of the lifting arm 13. The first lifting sub-arm 131 has a hollow structure with an inner diameter greater than the outer diameter of the second lifting sub-arm 132, allowing the second lifting sub-arm 132 to be retracted into the first lifting sub-arm 131. One end of the traction element 333 passes through the first lifting sub-arm 131 and is fixed to the second lifting sub-arm 132, enabling the traction element 333 to provide traction to move the second lifting sub-arm 132 relative to the first lifting sub-arm 131 vertically along the Z-axis direction.

In an embodiment, the moving member 12 is provided with an accommodating cavity in which the third electric assist member 33 is mounted. This concealed installation results in a cleaner appearance of the moving member 12 and also provides protection to the third electric assist member 33.

As shown in FIG. 11, in an embodiment, the fourth electric assist member 34 is housed within the rotating arm 14 so as to drive the rotation of the rotating arm 14 relative to the second end of the lifting arm 13 about the Z-axis direction. This integrated arrangement of the fourth electric assist member 34 within the rotating arm 14 allows for a concealed

installation, resulting in a cleaner and more streamlined appearance.

The fourth electric assist member 34 includes a fourth drive motor 341, a first helical gear 342, and a second helical gear 343. The fourth drive motor 341 is fixedly mounted inside the rotating arm 14, with its output shaft fixed to the first helical gear 342. The second helical gear 343 is fixed to the second end of the lifting arm 13. The first helical gear 342 and the second helical gear 343 are in mesh. The central axis of the second helical gear 343 is parallel to the Z-axis direction, while the central axis of the first helical gear 342 intersects with that of the second helical gear 343. Through the meshing transmission of the first helical gear 342 and the second helical gear 343, the fourth drive motor 341 drives the rotating arm 14 to rotate relative to the second end of the lifting arm 13 about the Z-axis direction.

Optionally, the mechanism may be configured with two first helical gears 342. One first helical gear 342 is fixed to the output shaft of the fourth drive motor 341, while the other is mounted inside the rotating arm 14 via a transmission shaft that is coaxial with the output shaft of the fourth drive motor 341. The two first helical gears 342 are arranged on both sides of the second helical gear 343 and are engaged with the second helical gear 343. Among the two first helical gears 342, the one connected to the fourth drive motor 341 serves as the driving gear, and the other as the driven gear. This dual-gear configuration enhances the stability of the meshing transmission between the first helical gears 342 and the second helical gear 343. Alternatively, a single first helical gear 342 meshing with the second helical gear 343 can also enable the rotating arm 14 to rotate relative to the second end of the lifting arm 13 about the Z-axis.

The first helical gear 342 and the second helical gear 343 may be configured as helical gears intersecting at 90°, with their central axes perpendicular to each other. This arrangement allows the fourth drive motor 341 to be mounted horizontally, thereby simplifying its installation and fixation.

In other embodiments, the first helical gear 342 and the second helical gear 343 may also intersect at other angles. For example, the central axes of the first helical gear 342 and the second helical gear 343 may form an angle of 80°. In such a configuration, the fourth drive motor 341 is oriented at a corresponding angle for installation, which can likewise enable the rotating arm 14 to rotate relative to the second end of the lifting arm 13 about the Z-axis.

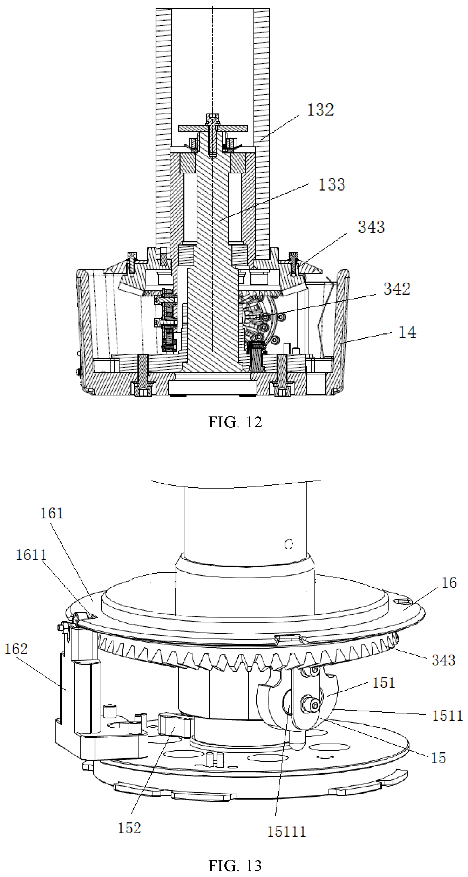

As shown in FIG. 12, in an embodiment, the rotating arm 14 has a hollow structure The fourth drive motor 341, the first helical gear 342, and at least a portion of the second helical gear 343 are enclosed within the rotating arm 14. This configuration allows the fourth electric assist member 34 to be concealed within the rotating arm 14, reducing the space occupied by the fourth electric assist member 34 and minimizing the overall dimensions of the X-ray generation device. It also provides protection to the fourth electric assist member 34 by shielding it from external interference.

As shown in FIG. 12, in an embodiment, the second helical gear 343 is of a hollow structure. The second end of the lifting arm 13 is rotatably connected to the rotating arm 14 via the first rotating shaft 133, and the first rotating shaft 133 extends through the central portion of the second helical gear 343. The lower end of the first rotating shaft 133 is fixed to the rotating arm 14, while the upper end of the first rotating shaft 133 is rotatably connected to the second end of the lifting arm 13 by means of a bearing. The first rotating shaft 133 functions to provide both connection and load-bearing support, ensuring that the cantilevered load from the lifting arm 13 is supported by the first rotating shaft 133. In this arrangement, the first helical gear 342 and the second helical gear 343 of the fourth electric assist member 34 are responsible solely for transmitting rotational drive and are free from gravitational loads, thereby ensuring the driving accuracy of the fourth electric assist member 34.

As shown in FIGS. 11 and 13, in an embodiment, the suspension assembly 10 further comprises a first limiting structure 15 arranged between the second end of the lifting arm 13 and the rotating arm 14. The first limiting structure 15 serves to restrict the range of the rotation of the rotating arm 14 relative to the second end of the lifting arm 13 about the Z-axis direction to an angular range of -180° to +180°, including the two boundary values of ±180°. This enables the control head to rotate through a full 360° without dead angles and to be accurately stopped at the same position in both forward and reverse directions.

The first limiting structure 15 includes a first limiting member 151 and a second limiting member 152. The first limiting member 151 is mounted on the second end of the lifting arm 13, and the second limiting member 152 is secured to the rotating arm 14. The first limiting member 151 is positioned along the circumferential rotation path of the second limiting member 152. When the rotating arm 14 rotates relative to the lifting arm 13 about the X-axis direction and reaches the limit position, the first limiting member 151 engages with the second limiting member 152, thereby restricting the rotational range of the rotating arm 14 about the Z-axis direction to -180° - +180°.

To prevent the circumferential thickness of the first limiting member 151 and the second limiting member 152 from restricting the rotational range of the rotating arm 14 about the Z-axis direction, the first limiting member 151 is configured as a swinging structure. The swinging motion of the first limiting member 151 compensates for its own circumferential thickness.

The first limiting member 151 may include a swinging member 1511 and an angle limiting member 1512. One end of the swinging member 1511 is swingably connected to the second end of the lifting arm 13, while the other end of the swinging member 1511 forms a limiting end that blockingly abuts against the second limiting member 152. The angle limiting member 1512 is arranged on the swinging path of the swinging member 1511 and serves to restrict the swing angle of the swinging member 1511, thereby limiting the rotational travel of the rotating arm 14 about the Z-axis direction to the

positions of -180° and 180°. Specifically, the swing angle of the swinging member 1511 is set to a specific value such that the spatial clearance created by the swinging motion of the swinging member 1511 equals the circumferential space occupied by the limiting end of the swinging member 1511. In this way, the swinging action of the swinging member 1511 compensates for the influence of its own circumferential thickness on the angular limitation.

Specifically, the swinging member 1511 may be configured as an annular structure. A first arc-shaped groove or a first arc-shaped hole 15111 is formed in the central portion of the swinging member 1511. The angle limiting member 1512 is fixed to the lifting arm 13, with a portion of it extending into the first arc-shaped groove or the first arc-shaped hole 15111. The first arc-shaped groove or the first arc-shaped hole 15111 has a predefined arc length to restrict the swing angle of the swinging member 1511. The swing angle of the swinging member 1511 is limited when the angle limiting member 1512 contacts either end of the first arc-shaped slot or aperture 15111.

In other embodiments, the swing member 1511 may be provided with a protrusion, and the angle limiting member 1512 is provided with a first arc-shaped groove or a first arc-shaped hole 15111. A portion of the protrusion of the swing member 1511 is disposed within the first arc-shaped groove or the first arc-shaped hole 15111, which can also limit the swing angle of the swing member 1511.

In other embodiments, the second limiting member 152 is configured as a swingable structure. By configuring the swinging motion of the second limiting member 152, the influence of the circumferential thickness of the first limiting member 151 and the second limiting member 152 on the rotational range of the rotating arm 14 about the Z-axis may be offset, thereby likewise enabling the rotational range of the rotating arm 14 about the Z-axis to be limited to -180° - +180°.

As shown in FIG. 13, in an embodiment, the suspension assembly 10 further includes a positioning structure 16 arranged between the second end of the lifting arm 13 and the rotating arm 14. The positioning structure 16 indexes the rotational angle of the rotating arm 14 about the Z-axis. A plurality of such positioning structures 16 may be provided, enabling the rotating arm 14 to be indexed at multiple different angular positions. For example, with four positioning structures 16 uniformly distributed around a circle at 90° intervals, the rotating arm 14 can be positioned at 0°, 90°, 180°, and 270°. The positioning structure 16 may also provide tactile feedback to indicate successful engagement to users, thereby enabling the user experience.

The positioning structure 16 may include a first positioning member 161 and a second positioning member 162. One of the first positioning member 161 and the second positioning member 162 is mounted on the second end of the lifting arm 13, and the other is mounted on the rotating arm 14. The first positioning member 161 is configured as an annular structure provided with a plurality of positioning holes 1611 along one of its circumferences. The second positioning member 162 is provided with a retractable elastic part 1621 at its end, which is engageable with the positioning holes 1611 to index the rotational angle of the rotating arm 14. The elastic part 1621 may comprise a ball bearing connected to a spring. The spring force urges the ball bearing into the positioning holes 1611 to achieve positioning. When sufficient force is applied, either manually or by the electric assist member, the ball bearing is retracted against the spring force, disengaging from the positioning holes 1611 and allowing the rotating arm 14 to continue its rotation about the Z-axis.

In other embodiments, the positioning structure 16 may also be configured as two magnetically opposite blocks. Positioning of the rotating arm 14 at a specific angle is achieved through magnetic attraction between these blocks.

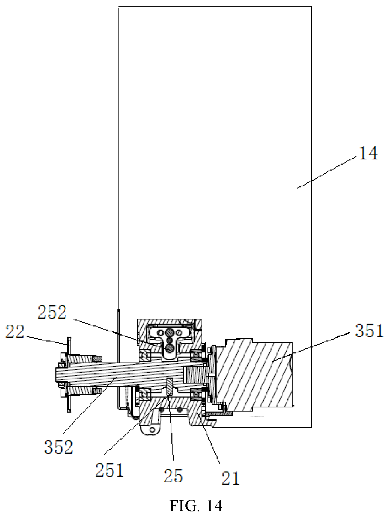

As shown in FIG. 14, in an embodiment, the fifth electric assist member 35 is disposed between the connection base 21 and the bracket 22. The fifth electric assist member 35 drives the bracket 22 to rotate relative to the connection base 21 about the first direction. The first direction is perpendicular to the length direction of the rotating arm 14. In the positional state shown in FIG. 1, the first direction is parallel to the Y-axis direction. As the rotating arm 14 rotates about the Z-axis, the first direction rotates accordingly.

The fifth electric assist member 35 includes a fifth drive motor 351 and a transmission shaft 352. The fifth drive motor 351 is mounted to the connection base 21. One end of the transmission shaft 352 is fixed to the output shaft of the fifth drive motor 351, and the other end is fixed to the bracket 22. The transmission shaft 352 is parallel to the first direction. The fifth drive motor 351 drives the bracket 22 to rotate relative to the connection base 21 along the first direction via the transmission shaft 352.

The connection base 21 may have a hollow structure. The fifth drive motor 351 and a portion of the transmission shaft 352 may be disposed within the connection base 21, thereby providing a concealed arrangement for the fifth electric assist member 35. This configuration reduces the space occupied by the fifth electric assist member 35 and provide hidden protection for it.

In other embodiments, the fifth drive motor 351 may be mounted to the bracket 22 to drive the bracket 22 to rotate relatively to the connection base 21 about the first direction.

With reference to FIG. 14, in an embodiment, the head assembly 20 further includes a second limiting structure 25 disposed between the connection base 21 and the bracket 22. The second limiting structure 25 limits the rotational angle range of the bracket 22 about the first direction, i.e., the rotational angle range of the control head 23 about the first direction, thereby preventing excessive rotation of the control head 23 about the first direction.

The second limiting structure 25 includes a third limiting member 251 and two fourth limiting members 252. The third limiting member 251 is mounted to the transmission shaft 352. The two fourth limiting members 252 are mounted to

the connection base 21 and are arranged in the rotational path of the third limiting member 251. The two fourth limiting members 252 abuts against the third limiting member 251, respectively, thereby limiting the rotational angle range of the bracket 22 about the first direction from -140° to +140°.

The third limiting member 251 and the two fourth limiting members 252 may be protruding block-like or plate-like structures. They are arranged to blockingly abut against each other on a common circular path, thereby limiting the rotation of the bracket 22 about the first direction.

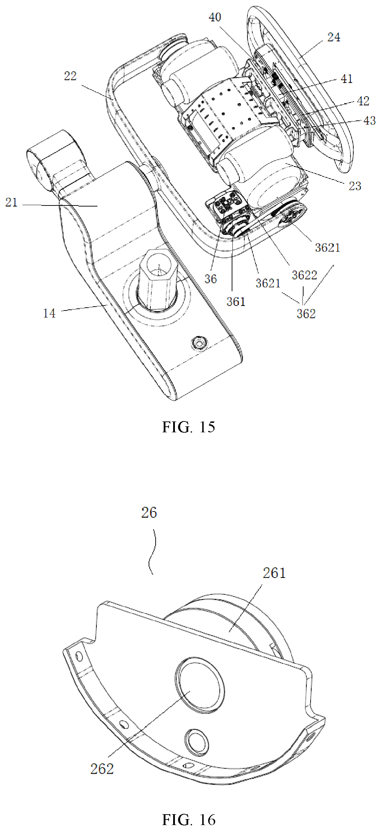

With reference to FIG. 15, in an embodiment, the sixth electric assist member 36 is disposed between the bracket 22 and the control head 23. The sixth electric assist member 36 drives the control head 23 to rotate relative to the bracket 22 about the second direction. The second direction is perpendicular to the first direction. In the positional state shown in FIG. 1, the second direction is parallel to the X-axis direction. As the rotating arm 14 rotates about the Z-axis direction, the second direction rotates accordingly.

The sixth electric assist member 36 includes a sixth drive motor 361 and a second transmission assembly 362. The sixth drive motor 361 is mounted to the control head 23 and is linked to the bracket 22 via the second transmission assembly 362. The sixth drive motor 361 drives the control head 23 to rotate about the second direction via the second transmission assembly 362. Mounting the sixth drive motor 361 to the control head 23 utilizes the space on one side of the control head 23 and simplifies the structure of the bracket 22.

The bracket 22 and the control head 23 are swingably connected by a second rotating shaft 221. The second rotating shaft 221 serves as a load-bearing swing to ensure stable rotation of the control head 23.

The second transmission assembly 362 includes two fourth drive pulleys 3621 and a fourth drive belt 3622. The output shaft of the sixth drive motor 361 is fixed to one of the fourth drive pulleys 3621. The other of the fourth drive pulleys 3621 is fixed to the bracket 22 and is coaxial with the second rotating shaft 221. The two fourth drive pulleys 3621 are coupled by the fourth drive belt 3622.

In other embodiments, the sixth drive motor 361 may be mounted to the bracket 22 to drive the control head 23 to rotate relative to the bracket 22 about the second direction.

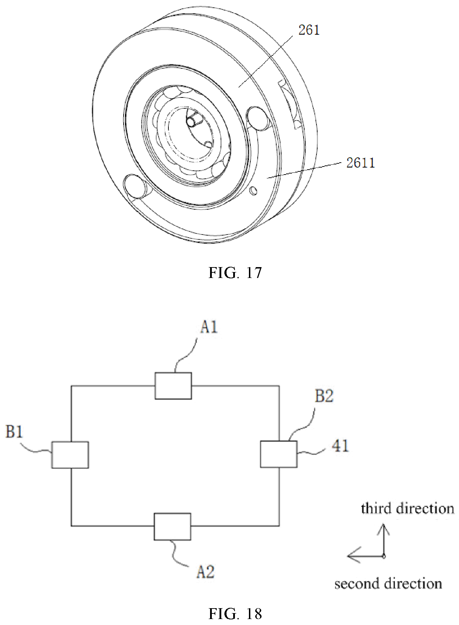

With reference to FIGS. 16 and 17, in an embodiment, the head assembly 20 further includes a third limiting structure 26. The third limiting structure 26 is disposed between the bracket 22 and the control head 23 to limit the rotational angle range of the control head 23 about the second direction from -10° to +90°. As the control head 23 is provided with a display, rotate the control head 23 to a downward-facing orientation is unnecessary and would be inconvenient for user operation.

The third limiting structure 26 includes a fifth limiting member 261 and a sixth limiting member 262. One of the fifth limiting member 261 and the sixth limiting member 262 is mounted to the bracket 22, and the other is mounted to the control head 23. The fifth limiting member 261 is provided with a second arc-shaped groove 2611 or a second arc-shaped hole. A portion of the sixth limiting member 262 is received in the second arc-shaped groove 2611 or the second arc-shaped hole, and the sixth limiting member 262 is movable along the second arc-shaped groove 2611 or the second arc-shaped hole. The second arc-shaped groove 2611 or the second arc-shaped hole is defined with a predetermined arc length, thereby limiting the rotational angle range of the control head 23 about the second direction from -10° to +90°.

The sixth limiting member 262 may be provided with a projecting pin or similar structure that is received in the second arc-shaped groove 2611 or the second arc-shaped hole. When the pin moves to either end of the second arc-shaped groove 2611 or the second arc-shaped hole, it limits the rotation of the control head 23 about the second direction.

In this embodiment, the fifth limiting member 261 may be integrally formed with the housing of the bracket 22 or the control head 23. By directly forming the second arc-shaped groove 2611 or the second arc-shaped hole on the housing, the rotational limitation of the control head 23 about the second direction is achieved.

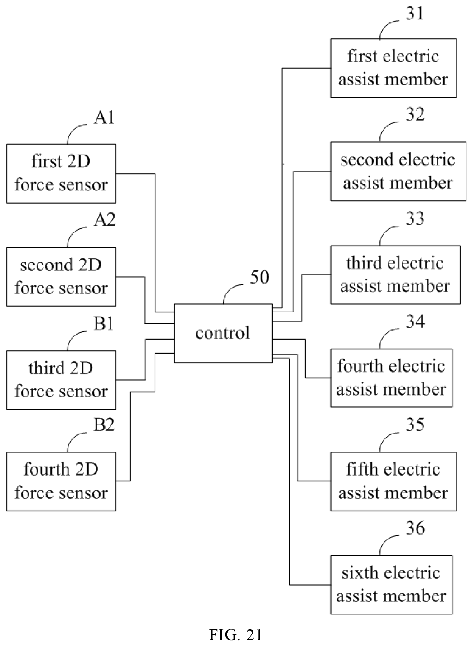

With reference to FIGS. 15, 18, 19, and 21, in an embodiment, the combined multi-dimensional force sensor 40 includes a force sensor group. The force sensor group includes at least four two-dimensional force sensors 41. This embodiment is described using four two-dimensional force sensors 41 as an example. The use of four two-dimensional force sensors 41 enables the detection of forces on the handle in six degrees of freedom using the minimum number of two-dimensional force sensors 41, thereby reducing sensor costs. In other embodiments, the combined multi-dimensional force sensor 40 may include a larger number of two-dimensional force sensors 41, such as six or eight two-dimensional force sensors 41, which can also detect the forces in six degrees of freedom of the handle.

In this embodiment, the four two-dimensional force sensors 41 are divided into a first group and a second group. The first group includes two oppositely arranged two-dimensional force sensors 41, and the second group includes two oppositely arranged two-dimensional force sensors 41. Here, "oppositely arranged" means that the two two-dimensional force sensors 41 are spaced apart from each other. The direction in which the two two-dimensional force sensors 41 in the first group are spaced apart is perpendicular to the direction in which the two two-dimensional force sensors 41 in the second group are spaced apart.

The two two-dimensional force sensors 41 in the first group detects forces on the handle 24 in the first direction and the second direction, and the two two-dimensional force sensors 41 in the second group detects forces on the handle 24 in the first direction and the third direction. The combination of the first group and the second group enables the

detection of three translational degrees of freedom (movement along the X-axis, Y-axis, and Z-axis) and three rotational degrees of freedom (rotation about the Z-axis, the first direction, and the second direction). Thus, the combination of four two-dimensional force sensors 41 enables six-degree-of-freedom (6-DOF). Since both the first group and the second group can detect respective forces in the first direction, when the handle 24 is rotated by 90° or 270°, the roles of the first group and the second group of sensors are interchanged. This results in the same two-dimensional force sensor configuration as in the initial state, thereby maintaining the 6-DOF detection capability for the control head 23.

Respective planes in which the first direction, the second direction and the third direction lie are not coplanar. The first direction, the second direction and the third direction are mutually perpendicular, and their respective planes are also mutually perpendicular. More preferably, the first direction, the second direction and the third direction are strictly perpendicular. This configuration simplifies the calculation of the accurate 6-DOF forces acting on the head 23 based on the detection signals from the two-dimensional force sensors 41.

In other embodiments, the first direction, the second direction and the third direction may be approximately perpendicular to each other. By incorporating a compensation algorithm for the angular deviation, the accurate values of the 6-DOF forces acting on the head 23 can still be calculated based on the detection signals from the two-dimensional force sensors 41.

In this embodiment, the four two-dimensional force sensors 41 can be distributed in a quadrilateral configuration. This quadrilateral shape may be a planar quadrilateral or a three-dimensional quadrilateral, that is, it is a closed planar or three-dimensional figure formed by four line segments that are not collinear and are connected end to end in sequence. For example, the four two-dimensional force sensors 41 are distributed on the four sides of a rectangle. Specifically, the four two-dimensional force sensors 41 are located on the four sides of a rectangle that lies in the same plane. The plane of this rectangle is parallel to the plane where the main body portion of the handle 24 is located, and the line connecting the center of the main body portion of the handle 24 to the center of the rectangle coincides with or is parallel to the second direction. This configuration enables the four two-dimensional force sensors 41 to correspond to the four sides of the main body portion of the handle 24. When a medical operator holds the four sides of the main body portion of the handle 24, the force can be accurately transmitted to the corresponding four two-dimensional force sensors 41. Such a layout is advantage for improving the detection accuracy of the four two-dimensional force sensors 41 and also helps to reduce the complexity of the algorithm.

In other embodiments, the four two-dimensional force sensors 41 in the first group may be disposed in a first plane, and the four two-dimensional force sensors 41 of the second group may be disposed in a second plane. Both the first plane and the second plane are parallel to the plane in which the main body portion of the handle 24 lies. The spacing direction of the four two-dimensional force sensors 41 in the first group is perpendicular to the spacing direction of the four two-dimensional force sensors 41 in the second group. This configuration also enables the 6-DOF detection.

In this embodiment, the four two-dimensional force sensors 41 may be respectively located at the centers of the four sides of a rectangle. That is, one two-dimensional force sensor 41 is disposed on the midline of each side of the rectangle, resulting in a symmetric distribution about a pair of coordinate axes. This pair of coordinate axes coincides with or is parallel to the coordinate axes defined by the X-axis and the Z-axis. Such a layout can greatly simplify the algorithm for calculating the six degrees of freedom from the signals of the four two-dimensional force sensors 41, and also facilitates ensuring installation accuracy.

In other embodiments, the four two-dimensional force sensors 41 may also be disposed at the four corners of the rectangle; that is, one two-dimensional force sensor 41 is disposed at each of the four corners. Alternatively, the two two-dimensional force sensors 41 in the first group are symmetrically arranged on two sides of the rectangle, and the two two-dimensional force sensors 41 in the second group are symmetrically arranged on the other two sides of the rectangle. Both of these layouts are also capable of 6-DOF detection, although the algorithm for resolving the sensed forces into the degrees of freedom is correspondingly more complex.

In this embodiment, employing a combination of four two-dimensional force sensors 41 to detect the six degrees of freedom required for the movement of the control head 23 significantly reduces the cost of the force sensors and enhances product competitiveness.

In the detection solution employing four two-dimensional force sensors 41, the correspondence between the detected force directions and the six degrees of freedom of motion is as shown in Table 1. When medical personnel rotate the handle 24, the two two-dimensional force sensors 41 in the first group and those in the second group are interchanged. The forces on the four two-dimensional force sensors 41 remain the same as before the rotation.

In Table 1, for the two two-dimensional force sensors 41 in the first group: the sensor 41 at the upper end is designated as the first two-dimensional force sensor A1, which can detect respective forces in both the first and second directions; the sensor 41 at the lower end is the second two-dimensional force sensor A2, which also detect the forces in the first and second directions. For the two two-dimensional force sensors 41 in the second group: the sensor 41 at the left end is the third two-dimensional force sensor B1, capable of detecting forces in the first and third directions; the sensor 41 at the right end is the fourth two-dimensional force sensor B2, which also detects forces in the first and third directions. The symbols "+" and "-" denote direction, while "/" indicates no force is detected. To explain the detection principle more clearly,

the positional state shown in FIG. 1 is taken as the initial state. In this state, the first direction corresponds to the Y-axis direction, the second direction corresponds to the X-axis direction, and the third direction corresponds to the Z-axis direction.

Table 1: Relationship Between Sensor Force Directions and Sensed Motion Directions

| Sensed Motion Direction | A1 | A2 | B1 | B2 |
|---|---|---|---|---|
| +X Translation | +X | +X | / | / |
| -X Translation | -X | -X | / | / |
| +Y Translation | +Y | +Y | +Y | +Y |
| -Y Translation | -Y | -Y | -Y | -Y |
| +Z Translation | / | / | +Z | +Z |
| -Z Translation | / | / | -Z | -Z |
| +X Rotation | / | / | +Y | -Y |
| -X Rotation | / | / | -Y | +Y |
| +Y Rotation | +Z | -Z | / | / |
| -Y Rotation | -Z | +Z | / | / |
| +Z Rotation | +Y | -Y | / | / |
| -Z Rotation | -Y | +Y | / | / |

In other embodiments, the force sensor group may include one or more types of sensors selected from one-dimensional, two-dimensional, and three-dimensional force sensors, while still being capable of detecting six-degree-of-freedom movements. The following arrangements are provided as examples:
The force sensor group includes eight one-dimensional force sensors. Two one-dimensional force sensors detects different directions are combined into a set, with each set being functionally analogous to one of the aforementioned two-dimensional force sensors 41. Accordingly, the eight one-dimensional force sensors can be arranged in a layout that mirrors the aforementioned installation pattern of the first group and the second group of two-dimensional force sensors 41, thereby achieving the detection of six-degree-of-freedom movement. This detection scheme using eight one-dimensional force sensors offers a relatively low sensor cost. However, the larger number of sensors increases the

difficulty of installation and calibration.

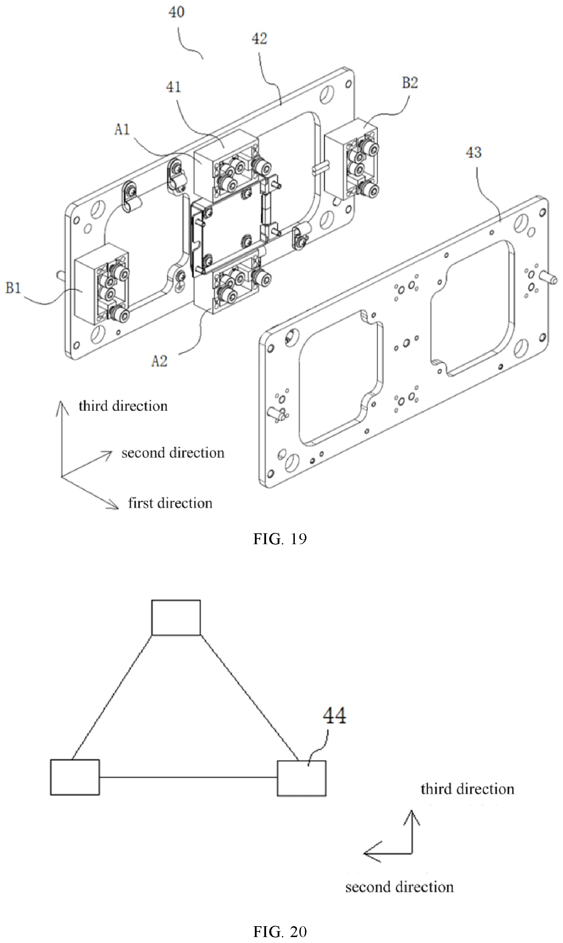

With reference to FIG. 20, the force sensor group includes at least three three-dimensional force sensors. Each three-dimensional force sensor may measure forces in the first, second, and third directions. The at least three three-dimensional force sensors may be arranged at the three vertices of a triangle. For example, arranging them at the three vertices of an isosceles triangle ensures equal spacing between the three three-dimensional force sensors, which facilitates simplification of the algorithm for resolving forces into degrees of freedom. Alternatively, the three three-dimensional force sensors may be disposed on the three sides of the aforementioned rectangle, which also enables detection of six-degree-of-freedom movement. A detection scheme employing three three-dimensional sensors offers relatively straightforward installation and calibration, albeit at a relatively higher sensor cost.

The force sensor group includes a six-dimensional force sensor. This sensor is capable of detecting forces in the first, second, and third directions, and outputs detection signals characterizing the movement of the handle 24 along the X, Y, and Z axes, as well as its rotation about the Z-axis direction, the first direction, and/or the second direction. In other words, a six-dimensional force sensor is functionally equivalent to a combination of four two-dimensional force sensors and can achieve six-degree-of-freedom direction. The six-dimensional sensor can be installed at any position between the handle 24 and the control head 23, allowing forces applied to the handle 24 to be transmitted to it. The use of a six-dimensional force sensor simplifies installation and calibration, although the cost of the sensor itself is relatively high.

In one embodiment, the combined multi-dimensional force sensor 40 can be configured as a modular structure. Four two-dimensional force sensors 41 are first assembled into a single module, which is then installed between the control head 23 and the handle 24. This allows for the calibration of the four two-dimensional force sensors 41 prior to the final assembly, thereby reducing the difficulty of both installation and calibration. The calibration of the four two-dimensional force sensors 41 here refers to the following: After the force sensors are fixed in place, the mounting screws apply a preload to them. Inevitable installation errors occur during the mounting of the four two-dimensional force sensors 41, and varying degrees of screw tightness result in different preloads being applied to the four two-dimensional force sensors 41. Consequently, the internal stress of the four two-dimensional force sensors 41 must be calibrated before formal detection begins. Algorithmic compensation is then used to eliminate the force error of the four two-dimensional force sensors 41 induced by these installation preloads, enabling the four two-dimensional force sensors 41 to measure the direction and magnitude of applied forces with greater accuracy.

The combined multi-dimensional force sensor 40 may include a first fixed frame 42 and a second fixed frame 43, which may be configured as rectangular plates. The force sensor group is mounted between the first fixed frame 42 and the second fixed frame 43. The four two-dimensional force sensors 41 are disposed on a rectangular mounting surface defined between these two frames. Together, the first fixed frame 42, the force sensor group, and the second fixed frame 43 form a sandwich-like, three-layer structure. The first fixed frame 42 and the second fixed frame 43 secure the four two-dimensional force sensors 41 via fasteners such as screws.

The first fixed frame 42 is fixed to the control head 23, and the second fixed frame 43 is fixed to the connecting portion of the handle 24. A force applied to the main body portion of the handle 24 is transmitted sequentially through the connecting portion of the handle 24 and the second fixed frame 43 to the force sensor group.

The force sensor group may be rigidly connected to the first fixed frame 42 and the second fixed frame 43, forming a rigid connection. The advantage of this rigid configuration is that the second fixed frame 43 remains stationary relative to the first fixed frame 42, meaning the handle 24 is fixed in position relative to the control head 23, which prevents wobbling.

In other embodiments, a movement clearance may exist between the force sensor group and one or both of the first fixed frame 42 and the second fixed frame 43, forming a flexible connection. The advantage of this flexible connection is that the force sensor are not subjected to high clamping forces during installation, which preserves their full range of force measurement. Furthermore, a damping element, such as a spring, may be disposed within this movement clearance to eliminate any wobbling of the handle 24 relative to the control head 23.

In other embodiments, the combined multi-dimensional force sensor 40 may omit the first fixed frame 42 and the second fixed frame 43. In this case, the force sensor group is installed directly between the control head 23 and the handle 24. After the multiple force sensors are calibrated on the fully assembled device, the system can still detect the force applied to the handle 24, and generates detection signals from which the six degrees of freedom of motion can be derived.

In one embodiment, the connecting portion of the handle 24 has a rectangular structure. This rectangular connecting portion is connected to the second fixed frame 43. The external contour of the connecting portion is identical or similar to that of the first fixed frame 42 and the second fixed frame 43, resulting in the outer contours of the first fixed frame 42 and the second fixed frame 43 being aligned with the outer contour of the rectangular connecting portion. This align-fit connection is more aesthetically pleasing and allows the combined multi-dimensional force sensor 40 to be installed in a concealed manner between the handle 24 and the control head 23.

In other embodiments, a recessed mounting slot may be provided on the connecting portion of the handle 24 or the control head 23. The combined multi-dimensional force sensor 40 is installed in this slot, thereby achieving a concealed effect and enhancing the aesthetics.

In an embodiment, an X-ray generation device is provided. This X-ray generation device includes the suspension

assembly 10 and the head assembly 20 from any of the aforementioned embodiments, but excludes the electric assist assembly 30 and the combined multi-dimensional force sensor 40. The control head 23 is capable of movement along the X-axis, movement along the Y-axis, vertical movement along the Z-axis, rotation about the Z-axis, rotation about the first direction, and rotation about the second direction. Medical personnel can operate the handle 24 to move, lift, and rotate the control head 23. Possessing six degrees of freedom of movement, the control head 23 is more flexible and can meet the imaging needs of multiple body parts of a patient in different postures, such as standing, lying flat, and sitting.

In one embodiment, an X-ray generation device is provided. The X-ray generation device includes the suspension assembly 10 and the head assembly 20 from any of the preceding embodiments. This X-ray generation device also includes an electric assist assembly 30 and a combined multi-dimensional force sensor 40. This X-ray generation device does not include the electric assist assembly 30 and the combined multi-dimensional force sensor 40. The control head 23 is capable of movement along the X-axis, movement along the Y-axis, rise and vertical movement along the Z-axis, rotation about the Z-axis, rotation about the first direction, and rotation about the second direction. By operating the handle 24, medical staff can move, lift, and rotate the control head 23. With six degrees of freedom of movement, the control head 23 offers greater flexibility, enabling it to meet the imaging requirements of multiple body parts of a patient in different postures, such as standing, lying flat, and sitting.

The electric assist assembly 30 may include one or more of the electric assist members described in the aforementioned embodiments to provide electric assist for one or more degrees of freedom of the control head 23. For example, the electric assist assembly 30 may include the first electric assist member 31, the second electric assist member 32, and the third electric assist member 33. The first electric assist member 31 drives the control head 23 to move along the X-axis, the second electric assist member 32 drives the control head 23 to move along the Y-axis, and the third electric assist member 33 drives the control head 23 to lift along the Z-axis. The rotation of the control head 23 about the Z-axis, about the first direction, and about the second direction may be performed manually. The control head 23 carries a relatively large load during translational movement along the X-axis, movement along the Y-axis, and movement along the Z-axis, where the benefit of electric assist is most significant, thereby improving operational convenience for medical personnel. In contrast, the load of the control head 23 during rotational movement about the Z-axis, about the first direction, and about the second direction is relatively small, making manual operation less burdensome. Therefore, this semi-electric assist and semi-manual driving method offers improved convenience in operating the control head 23 compared to a purely manual driving, saving time and effort.

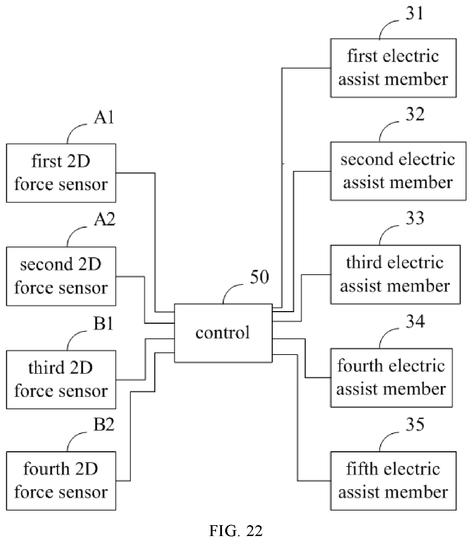

With reference to FIG. 22, in an embodiment, an X-ray generation device is provided. This X-ray generation device includes the suspension assembly 10, the electric assist assembly 30, and the combined multi-dimensional force sensor 40 from any of the preceding embodiments. This X-ray generation device further includes the head assembly 20. The head assembly 20 in this embodiment differs from those described previously in that its control head 23 is fixedly connected to the bracket 22. Consequently, the control head 23 in this embodiment possesses five degrees of freedom of movement: translation along the X-axis, translation along the Y-axis, vertical movement along the Z-axis, rotation about the Z-axis, and rotation about the first direction. Configuring the control head 23 with five degrees of freedom of movement meets the imaging needs for standing and supine patients, is applicable to most usage scenarios, and offers the advantage of relatively low cost.

The electric assist assembly 30 includes the first electric assist member 31, the second electric assist member 32, the third electric assist member 33, the fourth electric assist member 34, and the fifth electric assist member 35 described in the aforementioned embodiments. This configuration provides electric assist for the control head 23 to move along the X-axis, move along the Y-axis, move vertically along the Z-axis, rotate about the Z-axis, and rotate about the first direction. In other words, it achieve full electric assist for the five degrees of freedom of movement of the control head 23.

In other embodiments, the electric assist assembly 30 may include one or more of the electric assist members described in the above embodiments to provide electric assist for one or more degrees of freedom of the control head 23. For example, the electric assist assembly 30 may include the first electric assist member 31, the second electric assist member 32, and the third electric assist member 33. The first electric assist member 31 drives the control head 23 to move along the X-axis direction, the second electric assist member 32 drives the control head 23 to move along the Y-axis direction, and the third electric assist member 33 drives the control head 23 to move vertically along the Z-axis direction. Rotation of the control head 23 about the Z-axis direction and about the first direction is performed manually. The control head 23 presents a relatively high load during translational movement along the X-axis direction, along the Y-axis direction, and along the Z-axis direction, where the addition of electric assist provides significant benefits by improving operational convenience for medical personnel. In contrast, the load during rotational movement of the control head 23 about the Z-axis direction and about the first direction is relatively low, making direct manual drive less burdensome. Therefore, adopting a semi-electric assist and semi-manual driving method offers improved convenience in operating the control head 23 compared to a purely manual system, saving time and effort.

Provided in one embodiment is an X-ray imaging system including a flat panel detector and the X-ray generation device according to any of the preceding embodiments.

The flat panel detector is a free (unfixed) component that can be placed at different locations to collect X-rays

passing through a patient. The flat panel detector can be placed at a first imaging position that is detached from the cassette that stores it. The first imaging position is a free position and is placed according to the patient's posture. For example, when the patient is sitting in a wheelchair, the flat panel detector can be placed on the wheelchair at the position corresponding to the patient's anatomy for imaging. The flat panel detector can also be placed at a second imaging position where it is stored within the cassette. The second imaging position is a fixed position, which is a conventional position. When the patient is standing at an imaging column or lying on an imaging bed, the flat panel detector can be placed in the fixed cassette.

The six-degree-of-freedom movement of the control head 23 of the X-ray generation device, combined with the detachable flat panel detector that can be freely positioned, enables imaging of patients in both conventional postures (e.g., standing, lying down) and unconventional postures and positions. This configuration offers a wide range of applications and high compatibility.

The X-ray generation device is further provided with an electric assist member. This member provides electric assistance for the movement of the control head 23 within its operational space, greatly facilitating medical staff in aligning the control head 23 with the patient's region to be examined. This not only improves imaging efficiency but also enables more accurate and stable positioning of the control head 23, thereby enhancing the quality of the captured images.

The specific examples employed above to illustrate the present invention are for the purpose of facilitating understanding and are not intended to limit the invention. Those skilled in the art to which the present invention pertains, without departing from the basic principles of the present invention, may make various simple deductions, modifications or substitutions.

## Claims

**1.** An X-ray generation device, comprising:

a suspension assembly, comprising a first guide rail, a second guide rail, a moving member, a lifting arm, and a rotating arm, wherein the first guide rail extends along an X-axis direction, the second guide rail extends along a Y-axis direction, the second guide rail is movably connected to the first guide rail and is movable relative to the first guide rail along the X-axis direction, the moving member is movably connected to the second guide rail and is movable relative to the second guide rail along the Y-axis direction, the lifting arm has a first end and a second end that are opposite to each other, the first end of the lifting arm is connected to the moving member, and the second end of the lifting arm is liftable along a Z-axis direction, the rotating arm is connected to the second end of the lifting arm and is rotatable about the Z-axis direction;

a head assembly, comprising a connection base, a bracket, a control head and a handle, wherein the connection base is connected to the rotating arm, and the bracket is rotatably connected to the connection base and is rotatable relative to the connection base about a first direction, the control head generates X-rays and emits them to a region to be examined, the control head is rotatably connected to the bracket and is rotatable relative to the bracket about a second direction, the handle is connected to the control head and is operable by a user to effectuate the movement, lifting and/or rotation;

an electric assist assembly, comprising a first electric assist member, a second electric assist member, a third electric assist member, a fourth electric assist member, a fifth electric assist member, and a sixth electric assist member, wherein the first electric assist member is connected to the second guide rail so as to drive the second guide rail to move along the X-axis direction, the second electric assist member is connected to the moving member so as to drive the moving member to move along the Y-axis direction, the third electric assist member is connected to the lifting arm so as to drive the lifting arm to lift along the Z-axis direction, the fourth electric assist member is connected to the rotating arm so as to drive the rotating arm to rotate about the Z-axis direction, the fifth electric assist member is connected to the bracket so as to drive the bracket to rotate relative to the connection base about the first direction, the sixth electric assist member is connected to the control head so as to drive the control head to rotate relative to the bracket about the second direction;

a combined multi-dimensional force sensor, connected to the handle and detecting a force applied to the handle and generating corresponding detection signals; and

a controller, in signal communication with the combined multi-dimensional force sensor, the first electric assist member, the second electric assist member, the third electric assist member, the fourth electric assist member, the fifth electric assist member, and the sixth electric assist member, wherein the controller obtains the detection signals, generates corresponding control signals, and sends the control signals to one or more of the first electric assist member, the second electric assist member, the third electric assist member, the fourth electric assist member, the fifth electric assist member, and the sixth electric assist member, so as to control the movement, lift, and/or rotation of the control head.

**2.** The X-ray generation device according to claim 1, wherein:

the first electric assist member comprises a first drive motor, a first drive pulley, and a first drive belt, wherein the first drive motor and the first drive pulley are mounted on the second guide rail, the first drive belt is mounted on the first guide rail along the X-axis direction, an output shaft of the first drive motor is fixedly connected to the first drive pulley, the first drive pulley is engaged with the first drive belt, and the first drive motor drives the first drive pulley to travel relative to the first drive belt along the X-axis direction, thereby causing the second guide rail to move relative to the first guide rail along the X-axis direction;
and/or,
the second electric assist member comprises a second drive motor, a second drive pulley, and a second drive belt, wherein the second drive motor and the second drive pulley are mounted on the moving member, the second drive belt is mounted on the second guide rail along the Y-axis direction, an output shaft of the second drive motor is fixedly connected to the second drive pulley, the second drive pulley is engaged with the second drive belt, the second drive motor drives the second drive pulley to travel relative to the second drive belt along the Y-axis direction, thereby causing the moving member to move relative to the second guide rail along the Y-axis direction.

**3.** The X-ray generation device according to claim 1, wherein:

the third electric assist member comprises a third drive motor, a first transmission assembly, and a traction element that are mounted on the moving member;
the third drive motor is connected to the traction element via the first transmission assembly;
the traction element extends to connect to the second end of the lifting arm; and
the third drive motor drives, via the first transmission assembly, the traction element to lift, thereby driving the second end of the lifting arm to lift along the Z-axis direction.

**4.** The X-ray generation device according to claim 3, wherein:

the first transmission assembly comprises two third drive pulleys, a third drive belt, and a roller shaft;
one of the third drive pulleys is fixedly connected to an output shaft of the third drive motor, and the other of the third drive pulleys is fixedly connected to the roller shaft;
the two third drive pulleys are engaged with the third drive belt; and
the traction element is a traction rope wound around the roller shaft, with one end of the traction rope fixedly connected to the roller shaft, and the other end of the traction rope fixedly connected to the second end of the lifting arm.

**5.** The X-ray generation device according to claim 3, wherein:

the lifting arm at least comprises a first lifting sub-arm and a second lifting sub-arm;
the first lifting sub-arm and the second lifting sub-arm are movably connected for lifting along the Z-axis direction;
an end of the first lifting sub-arm away from the second lifting sub-arm is defined as the first end of the lifting arm;
an end of the second lifting sub-arm away from the first lifting sub-arm is defined as the second end of the lifting arm;
the first lifting sub-arm has a hollow structure; and
an end of the traction element extends through the first lifting sub-arm so as to be fixedly connected to the second lifting sub-arm.

**6.** The X-ray generation device according to claim 1, wherein:

the fourth electric assist member comprises a fourth drive motor, a first helical gear, and a second helical gear;
the fourth drive motor is mounted on the rotating arm;
an output shaft of the fourth drive motor is fixedly connected to the first helical gear;
the second helical gear is fixedly connected to the second end of the lifting arm;
the first helical gear is meshed with the second helical gear;
a central axis of the second helical gear is parallel to the Z-axis direction; and
a central axis of the first helical gear intersects with the central axis of the second helical gear.

**7.** The X-ray generation device according to claim 6, wherein:
the central axis of the first helical gear is perpendicular to the central axis of the second helical gear.

**8.** The X-ray generation device according to claim 6, wherein:

the rotating arm has a hollow structure; and
the fourth drive motor, the first helical gear and at least a portion of the second helical gear are disposed within the rotating arm.

**9.** The X-ray generation device according to claim 6, wherein:

the second helical gear has a hollow structure;
the second end of the lifting arm is rotatably connected to the rotating arm by a first rotating shaft; and
the first rotating shaft extends through a central portion of the second helical gear.

**10.** The X-ray generation device according to claim 1, wherein:

the suspension assembly further comprises a first limiting structure;
the first limiting structure comprises a first limiting member and a second limiting member;
the first limiting member is mounted on the second end of the lifting arm;
the second limiting member is arranged on the rotating arm;
the first limiting member is mounted on a circular path along which the second limiting member rotates; and
the first limiting member abuts against the second limiting member, so as to limit an angular range of the rotation of the rotating arm about the Z-axis direction to -180° - +180°.

**11.** The X-ray generation device according to claim 10, wherein:

the first limiting member comprises a swing member and an angle limiting member;
one end of the swing member is rotatably connected to the second end of the lifting arm;
the other end of the swing member, defined as a limiting end, blockingly abuts against the second limiting member;
the angle limiting member is arranged on a swing path of the swing member; and
the angle limiting member limits a swing angle of the swing member, thereby limiting maximum rotational positions of the rotating arm about the Z-axis direction at -180° and +180°.

**12.** The X-ray generation device according to claim 11, wherein:

a central portion of the swing member is provided with a first arc-shaped groove or a first arc-shaped hole;
a portion of the angle limiting member is disposed in the first arc-shaped groove or the first arc-shaped hole; and
the first arc-shaped groove or the first arc-shaped hole has a predetermined arc length for limiting the swing angle of the swing member.

**13.** The X-ray generation device according to claim 1, wherein:

the suspension assembly further comprises a positioning structure;
the positioning structure comprises a first positioning member and a second positioning member;
one of the first positioning member and the second positioning member is mounted on the second end of the lifting arm, and the other of the first positioning member and the second positioning member is mounted on the rotating arm;
the first positioning member is a ring-shaped structure provided with a plurality of positioning holes along a circumference thereof; and
an end portion of the second positioning member is provided with a retractable elastic portion that is engageable with the positioning holes, so as to position a rotational angle of the rotating arm.

**14.** The X-ray generation device according to claim 1, wherein:

the fifth electric assist member comprises a fifth drive motor and a transmission shaft;
the fifth drive motor is mounted on the connection base;
one end of the transmission shaft is fixedly connected to an output shaft of the fifth drive motor;
the other end of the transmission shaft is fixedly connected to the bracket; and
the transmission shaft is parallel to the first direction.

**15.** The X-ray generation device according to claim 14, wherein:

the head assembly further comprises a second limiting structure;
the second limiting structure comprises a third limiting member and two fourth limiting members;
the third limiting member is mounted on the transmission shaft;
the two fourth limiting members are mounted on the connection base;
the two fourth limiting members are arranged on a circular path along which the third limiting member rotates; and
the two fourth limiting members respectively abut against the third limiting member, so as to limit an angular range of the rotation of the bracket about the first direction to -140° - +140°.

**16.** The X-ray generation device according to claim 1, wherein:

the sixth electric assist member comprises a sixth drive motor and a second transmission assembly;
the sixth drive motor is mounted on the control head;
the sixth drive motor is connected to the bracket via the second transmission assembly; and
the sixth drive motor drives, via the second transmission assembly, the bracket to rotate about the second direction.

**17.** The X-ray generation device according to claim 16, wherein:

the bracket and the control head are rotatably connected via a second rotating shaft;
the second transmission assembly comprises two fourth drive pulleys and a fourth drive belt;
one of the fourth drive pulleys is fixedly connected to an output shaft of the sixth drive motor, and the other of the fourth drive pulleys is fixedly connected to the bracket and is arranged coaxially with the second rotating shaft; and
the two fourth drive pulleys are engaged with the fourth drive belt.

**18.** The X-ray generation device according to claim 1, wherein:

the head assembly further comprises a third limiting structure;
the third limiting structure comprises a fifth limiting member and a sixth limiting member;
one of the fifth limiting member and the sixth limiting member is mounted on the bracket, and the other of the fifth limiting member and the sixth limiting member is mounted on the control head;
the fifth limiting member is provided with a second arc-shaped groove or a second arc-shaped hole;
a portion of the sixth limiting member is engaged in the second arc-shaped groove or the second arc-shaped hole;
the sixth limiting member moves within the second arc-shaped groove or the second arc-shaped hole; and
the second arc-shaped groove or the second arc-shaped hole has a predetermined arc length for limiting an angular range of the rotation of the control head about the second direction to -10° - +90°.

**19.** The X-ray generation device according to claim 1, wherein:

the combined multi-dimensional force sensor comprises a force sensor group;
the force sensor group comprises one or more of: a one-dimensional force sensor, a two-dimensional force sensor, and a three-dimensional force sensor; and
the force sensor group detects respective forces applied to the handle in the first direction, the second direction and a third direction, and outputs the detection signals representing movement of the handle along the X-axis direction, movement of the handle along the Y-axis direction, movement of the handle along the Z-axis direction, rotation of the handle about the Z-axis direction, rotation of the handle about the first direction, and/or rotation of the handle about the second direction.

**20.** The X-ray generation device according to claim 19, wherein:

the force sensor group comprises at least four two-dimensional force sensors;
the at least four two-dimensional force sensors are divided into a first group and a second group;
the first group and the second group each comprises two two-dimensional force sensors disposed opposite to each other;
the two two-dimensional force sensors of the first group detect respective forces applied to the handle in the first direction and the second direction;
the two two-dimensional force sensors of the second group detect respective forces applied to the handle in the

first direction and the third direction; and
respective planes in which the first direction, the second direction and the third direction lie are not coplanar.

**21.** The X-ray generation device according to claim 20, wherein:

the at least four two-dimensional force sensors are arranged in a quadrilateral configuration;
the quadrilateral is a planar quadrilateral or a three-dimensional quadrilateral.

**22.** The X-ray generation device according to claim 21, wherein:

a plane defined by the quadrilateral is parallel to a plane defined by the handle;
the quadrilateral is a rectangle;
the two two-dimensional force sensors of the first group are symmetrically arranged on two sides of the rectangle, and the two two-dimensional force sensors of the second group are symmetrically arranged on other two sides of the rectangle;
or,
the four two-dimensional force sensors are respectively arranged at a center of each side of the rectangle;
or,
the four two-dimensional force sensors are respectively arranged at each corner of the rectangle.

**23.** The X-ray generation device according to claim 19, wherein:

the force sensor group comprises at least three three-dimensional force sensors;
the three three-dimensional force sensors are arranged at three vertices of a triangle;
a plane defined by the triangle is parallel to a plane defined by the handle;
the three-dimensional force sensors detect respective forces applied to the handle in the first direction, the second direction, and the third direction; and
respective planes in which the first direction, the second direction and the third direction lie are not coplanar.

**24.** The X-ray generation device according to claim 19, wherein:

the first direction, the second direction, and the third direction are mutually perpendicular; and
the first direction is perpendicular to a plane defined by the handle.

**25.** The X-ray generation device according to any one of claims 19 to 24, wherein:

the combined multi-dimensional force sensor further comprises a first fixed frame and a second fixed frame;
the force sensor group is mounted between the first fixed frame and the second fixed frame;
the first fixed frame is connected to the control head; and
the second fixed frame is connected to the handle.

**26.** The X-ray generation device according to claim 25, wherein:
the force sensor group is fixedly connected to the first fixed frame and the second fixed frame.

**27.** The X-ray generation device according to claim 25, wherein:

the force sensor group is connected to the first fixed frame and the second fixed frame;
there exists a movement clearance between the force sensor group and at least one of the first fixed frame and the second fixed frame; and
the force sensor group is movable within the clearance.

**28.** The X-ray generation device according to claim 25, wherein:

a connecting portion between the handle and the control head has a rectangular structure;
the rectangular structure is connected to the second fixed frame; and
outer contours of the first fixed frame and the second fixed frame are aligned with an outer contour of the rectangular structure.

**29.** The X-ray generation device according to claim 1, wherein:

the combined multi-dimensional force sensor comprises a six-dimensional force sensor;
the six-dimensional force sensor detects respective forces applied to the handle in the first direction, the second direction, and a third direction, and outputs the detection signals representing movement of the handle along the X-axis direction, movement of the handle along the Y-axis direction, movement of the handle along the Z-axis direction, rotation of the handle about the Z-axis direction, rotation of the handle about the first direction, and/or rotation of the handle about the second direction.

**30.** The X-ray generation device according to claim 1, wherein:

the second end of the lifting arm is rotatably connected to the rotating arm;
or, the second end of the lifting arm is rotatably connected to the first end thereof.

**31.** An X-ray generation device, comprising:

a suspension assembly, comprising a first guide rail, a second guide rail, a moving member, a lifting arm, and a rotating arm, wherein the first guide rail extends along an X-axis direction, the second guide rail extends along a Y-axis direction, the second guide rail is movably connected to the first guide rail and is movable relative to the first guide rail along the X-axis direction, the moving member is movably connected to the second guide rail and is movable relative to the second guide rail along the Y-axis direction, the lifting arm has a first end and a second end that are opposite to each other, the first end of the lifting arm is connected to the moving member, and the second end of the lifting arm is liftable relative to the first end along a Z-axis direction; the rotating arm is connected to the second end of the lifting arm and is rotatable relative to the first end of the lifting arm about the Z-axis direction; and
a head assembly, comprising a connection base, a bracket, a control head and a handle, wherein the connection base is connected to the rotating arm, and the bracket is rotatably connected to the connection base and is rotatable relative to the connection base about a first direction, the control head is configured to emit X-rays to a region to be examined, the control head is rotatably connected to the bracket and is rotatable relative to the bracket about a second direction, the handle is connected to the control head so as to be operated by a user to effectuate the movement, lifting, and/or rotation.

**32.** The X-ray generation device according to claim 31, further comprising:

an electric assist assembly, comprising at least one electric assist member, wherein the at least one electric assist member is connected to at least one of the second guide rail, the moving member, the lifting arm, the rotating arm, the bracket, and the control head; the at least one electric assist member drives at least one of: movement of the second guide rail relative to the first guide rail along the X-axis direction, movement of the moving member relative to the second guide rail along the Y-axis direction, lifting of the second end of the lifting arm relative to the first end along the Z-axis, rotation of the rotating arm relative to the lifting arm about the Z-axis, rotation of the bracket relative to the rotating arm about the first direction, and rotation of the control head relative to the bracket about the second direction;
a combined multi-dimensional force sensor, connected to the control head and the handle, wherein the combined multi-dimensional force sensor detects a force direction and a force magnitude of the handle, and generate corresponding detection signals; and
a controller, in signal communication with the combined multi-dimensional force sensor and the at least one electric assist member, wherein the controller obtains the detection signals, generates corresponding control signals, and sends the control signals to the at least one electric assist member, so as to control the movement, lifting, and/or rotation of the control head.

**33.** The X-ray generation device according to claim 32, wherein: the combined multi-dimensional force sensor comprises a force sensor group that comprises at least four two-dimensional force sensors, wherein:

the at least four two-dimensional force sensors are divided into a first group and a second group;
the first group and the second group each comprises two two-dimensional force sensors disposed opposite to each other;
the two two-dimensional force sensors of the first group detects forces applied to the handle in the first direction and the second direction;
the two-dimensional force sensors of the second group detects forces applied in the first direction and the third

direction; and
respective planes in which the first direction, the second direction and the third direction lie are not coplanar.

**34.** The X-ray generation device according to claim 33, wherein:

the combined multi-dimensional force sensor further comprises a first fixed frame and a second fixed frame;
the force sensor group is mounted between the first fixed frame and the second fixed frame;
the first fixed frame is connected to the control head; and
the second fixed frame is connected to the handle.

**35.** The X-ray generation device according to claim 34, wherein:

a connecting portion between the handle and the control head has a rectangular structure;
the rectangular structure is connected to the second fixed frame; and
outer contours of the first fixed frame and the second fixed frame are aligned with outer contour of the rectangular structure.

**36.** The X-ray generation device according to claim 33, wherein:

the first direction, the second direction, and the third direction are mutually perpendicular; and
the first direction is perpendicular to a plane defined by the handle.

**37.** An X-ray generation device, comprising:

a suspension assembly, comprising a first guide rail, a second guide rail, a moving member, a lifting arm, and a rotating arm, wherein the first guide rail extends along an X-axis direction, the second guide rail extends along a Y-axis direction, the second guide rail is movably connected to the first guide rail and is movable relative to the first guide rail along the X-axis direction, the moving member is movably connected to the second guide rail and is movable relative to the second guide rail along the Y-axis direction; the lifting arm has a first end and a second end that are opposite to each other, the first end of the lifting arm is connected to the moving member, the second end of the lifting arm is liftable relative to the first end along a Z-axis direction, the rotating arm is connected to the second end of the lifting arm, and the rotating arm is rotatable relative to the second end of the lifting arm about the Z-axis direction;
a head assembly, comprising a connection base, a bracket, a control head, and a handle, wherein the connection base is connected to the rotating arm, the bracket is rotatably connected to the connection base, the bracket is rotatable relative to the connection base about the first direction, the control head emits X-rays to a region to be examined and is connected to the bracket; and the handle is connected to the control head so as to be operated by a user to effectuate the movement, lifting, and/or rotation of the control head;
an electric assist assembly, comprising at least one electric assist member, wherein the at least one electric assist member is connected to at least one of the second guide rail, the moving member, the lifting arm, the rotating arm, and the bracket; the at least one electric assist member drives: movement of the second guide rail relative to the first guide rail along the X-axis, movement of the moving member relative to the second guide rail along the Y-axis direction, lifting of the second end of the lifting arm relative to the first end along the Z-axis direction, rotation of the rotating arm relative to the lifting arm about the Z-axis, and rotation of the bracket relative to the rotating arm about the Y-axis direction;
a combined multi-dimensional force sensor, connected to the control head and the handle combined multi-dimensional force sensor, wherein the combined multi-dimensional force sensor detects a force direction and a force magnitude of the handle and generate corresponding detection signals; and
a controller, in signal communication with the combined multi-dimensional force sensor and the at least one electric assist member, wherein the controller obtains the detection signals, generates corresponding control signals, and sends the control signals to the at least one electric assist member, so as to control the movement, lifting, and/or rotation of the control head.

**38.** The X-ray generation device according to claim 37, wherein:

the electric assist assembly comprises a first electric assist member, a second electric assist member, a third electric assist member, a fourth electric assist member, and fifth electric assist member; the first electric assist member is connected to the second guide rail so as to drive the second guide rail to move along the X-axis

direction; the second electric assist member is connected to the moving member so as to drive the moving member to move along the Y-axis direction; the third electric assist member is connected to the lifting arm so as to drive the second end of the lifting arm to lift relative to the first end along the Z-axis; the fourth electric assist member is connected to the rotating arm so as to drive the rotating arm to rotate relative to the lifting arm about the Z-axis; and the fifth electric assist member is connected to the bracket so as to drive the bracket to rotate relative to the rotating arm about the first direction;
the controller, in signal communication with the first electric assist member, the second electric assist member, third electric assist member, the fourth electric assist member, and the fifth electric assist member, obtains the detection signals, generates corresponding control signals, and sends the control signals to one or more of the first electric assist member, the second electric assist member, the third electric assist member, the fourth electric assist member, and the fifth electric assist member, so as to control the movement, lifting, and/or rotation of the control head.

**39.** The X-ray generation device according to claim 38, wherein the combined multi-dimensional force sensor comprises a force sensor group that comprises at least four two-dimensional force sensors; wherein:

the at least four two-dimensional force sensors are divided into a first group and a second group;
the first group and the second group each comprises two two-dimensional force sensor disposed opposite to each other;
the two-dimensional force sensors of the first group detect respective forces applied to the handle in the first direction and the second direction; and
the two two-dimensional force sensors of the second group detect respective forces in the first direction and the third direction.

**40.** The X-ray generation device according to claim 37, wherein:

the combined multi-dimensional force sensor further comprises a first fixed frame and a second fixed frame;
the force sensor group is mounted between the first fixed frame and the second fixed frame;
the first fixed frame is connected to the control head; and
the second fixed frame is connected to the handle.

**41.** The X-ray generation device according to claim 40, wherein:

a connecting portion between the handle and the control head has a rectangular structure;
the rectangular structure is connected to the second fixed frame; and
outer contours of the first fixed frame and the second fixed frame are aligned with an outer contour of the rectangular structure.

**42.** An X-ray imaging system, comprising:

the X-ray generation device according to any one of claims 1 to 41; and
a flat panel detector, configured to be placed at a first imaging position detached from a cassette that houses the flat panel detector, and further configured to be placed at a second imaging position housed within the cassette;
wherein the control head aligns the flat panel detector; the flat panel detector collects X-rays penetrating the region to be examined and generate corresponding imaging signals that are used for obtaining a captured X-ray image.

10
31
11
30
11
111
112
21
12
13
231
X-axis direction
Y-axis direction
Z-axis direction
14
22
23
20
24
second direction
first direction
third direction

FIG. 1

111
112
12
13
24
23
22
21
14
X-axis direction
Y-axis direction
Z-axis direction
second direction
first direction
third direction

FIG. 2

first direction
second direction
third direction
35
21
13
34
14
22
23
231
24

FIG. 3

first direction
second direction
third direction
25
13
34
14
24
23
22
21
35

FIG. 4

40
50
30
combined multi-dimensional force sensor
control
electric assist assembly

FIG. 5

30
31
112
111
third direction
first direction
second direction

FIG. 6

311
312
313
12

FIG. 7

323
321
322

FIG. 8

332
3321
3322
3321
333
3323
12
331

FIG. 9

111
12
112
131
22
23
24
132
14

FIG. 10

13
132
14
343
1511
15111
151
1512
342
341

FIG. 11

132
133
343
342
14

FIG. 12

161
1611
16
162
343
151
1511
15
152
15111

FIG. 13

14
252
22
351
352
251
25
21

FIG. 14

40
24
22
41
42
43
23
21
3621
36
3622
3621
361
14
362

FIG. 15

26
261
262

FIG. 16

261
2611

FIG. 17

A1
B2
B1
41
third direction
A2
second direction

FIG. 18

40
42
41
B2
A1
43
B1
A2
third direction
second direction
first direction

FIG. 19

44
third direction
second direction

FIG. 20

31
first electric
assist member
32
second electric
assist member
33
third electric
assist member
34
fourth electric
assist member
35
fifth electric
assist member
36
sixth electric
assist member
A1
first 2D
force sensor
A2
second 2D
force sensor
B1
third 2D
force sensor
B2
fourth 2D
force sensor
50
control

FIG. 21

31
first electric assist member
32
second electric assist member
33
third electric assist member
34
fourth electric assist member
35
fifth electric assist member
A1
first 2D force sensor
A2
second 2D force sensor
B1
third 2D force sensor
B2
fourth 2D force sensor
50
control

FIG. 22

## INTERNATIONAL SEARCH REPORT

International application No.
**PCT/CN2024/127069**

**A. CLASSIFICATION OF SUBJECT MATTER**

A61B6/00(2024.01)i

According to International Patent Classification (IPC) or to both national classification and IPC

**B. FIELDS SEARCHED**

Minimum documentation searched (classification system followed by classification symbols)

IPC: A61B6

Documentation searched other than minimum documentation to the extent that such documents are included in the fields searched

Electronic data base consulted during the international search (name of data base and, where practicable, search terms used)

CNABS, CNTXT: 移动, 升降, 旋转, 转动, 限位, 角度, 控制; VEN, USTXT, EPTXT, WOTXT: move, lift, descend, rotate, limit, angle, control.

**C. DOCUMENTS CONSIDERED TO BE RELEVANT**

| Category* | Citation of document, with indication, where appropriate, of the relevant passages | Relevant to claim No. |
|---|---|---|
| X | CN 109890290 A (SHIMADZU CORP.) 14 June 2019 (2019-06-14) description, paragraphs 61-158, and figures 1-12 | 1-9, 13-42 |
| Y | CN 109890290 A (SHIMADZU CORP.) 14 June 2019 (2019-06-14) description, paragraphs 61-158, and figures 1-12 | 10-12 |
| Y | CN 109451282 A (WATRIX.AI TECHNOLOGY (BEIJING) CO., LTD. et al.) 08 March 2019 (2019-03-08) description, paragraphs 89-113, and figures 1-9 | 10-12 |
| X | CN 111374685 A (SHIMADZU CORP.) 07 July 2020 (2020-07-07) description, paragraphs 22-68, and figures 1-5 | 1-9, 13-42 |
| X | KR 20140019273 A (SAMSUNG ELECTRONICS CO., LTD.) 14 February 2014 (2014-02-14) description, paragraphs 30-82, and figures 1-7 | 1-9, 13-42 |
| A | CN 1929785 A (KONINKL PHILIPS ELECTRONICS N.V.) 14 March 2007 (2007-03-14) entire document | 1-42 |

☐ Further documents are listed in the continuation of Box C.    ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 January 2025** | **25 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/127069**

| Patent document cited in search report | | | Publication date (day/month/year) | Patent family member(s) | | | Publication date (day/month/year) |
|---|---|---|---|---|---|---|---|
| CN | 109890290 | A | 14 June 2019 | WO | 2018042483 | A1 | 08 March 2018 |
| | | | | JP | 6610795 | B2 | 27 November 2019 |
| | | | | US | 2019239835 | A1 | 08 August 2019 |
| | | | | US | 10849582 | B2 | 01 December 2020 |
| | | | | JP | WO2018042483 | A1 | 24 June 2019 |
| | | | | CN | 109890290 | B | 18 April 2023 |
| CN | 109451282 | A | 08 March 2019 | CN | 109451282 | B | 20 October 2023 |
| CN | 111374685 | A | 07 July 2020 | JP | 2020103335 | A | 09 July 2020 |
| | | | | JP | 7119987 | B2 | 17 August 2022 |
| | | | | US | 2020205759 | A1 | 02 July 2020 |
| | | | | US | 11116460 | B2 | 14 September 2021 |
| | | | | CN | 111374685 | B | 28 May 2024 |
| KR | 20140019273 | A | 14 February 2014 | KR | 101662692 | B1 | 05 October 2016 |
| | | | | CN | 102551747 | A | 11 July 2012 |
| | | | | CN | 104856711 | A | 26 August 2015 |
| | | | | CN | 104856711 | B | 01 October 2019 |
| | | | | US | 2012087480 | A1 | 12 April 2012 |
| | | | | US | 8651740 | B2 | 18 February 2014 |
| | | | | KR | 20120035645 | A | 16 April 2012 |
| | | | | KR | 101417780 | B1 | 09 July 2014 |
| | | | | CN | 102551747 | B | 04 November 2015 |
| CN | 1929785 | A | 14 March 2007 | US | 2007140435 | A1 | 21 June 2007 |
| | | | | US | 7641391 | B2 | 05 January 2010 |
| | | | | JP | 2007527763 | A | 04 October 2007 |
| | | | | WO | 2005087105 | A2 | 22 September 2005 |
| | | | | PL | 1727466 | T3 | 30 September 2015 |
| | | | | EP | 1727466 | A2 | 06 December 2006 |
| | | | | EP | 1727466 | B1 | 03 December 2014 |
| | | | | EP | 1727466 | B8 | 21 January 2015 |
| | | | | ES | 2530682 | T3 | 04 March 2015 |
| | | | | CN | 100496398 | C | 10 June 2009 |
| | | | | US | 2013099448 | A1 | 25 April 2013 |

Form PCT/ISA/210 (patent family annex) (July 2022)